# EUROPEAN PATENT APPLICATION

(11) **EP 3 459 351 A1**
(43) Date of publication of application: **27.03.2019**
(21) Application number: 17799513.1
(22) Date of filing: 19.05.2017
(51) Int. Cl.: A01N 1/02, A61M 1/00

(54) **PERFUSION APPARATUS FOR LIVER FOR TRANSPLANTATION, AND LIVER ISOLATION METHOD AND LIVER TRANSPLANTATION METHOD USING SAID APPARATUS**

(30) Priority: 20.05.2016 JP 2016101124
(71) Applicant: SCREEN Holdings Co., Ltd., Kyoto 602-8585 (JP); Riken, Wako-shi, Saitama 351-0198 (JP)
(72) Inventor: KOBAYASHI Eiji, Kyoto-shi Kyoto 602-8585 (JP); TSUJI Takashi, Wako-shi Saitama 351-0198 (JP); ISHIKAWA Jun, Wako-shi Saitama 351-0198 (JP); NADAHARA Soichi, Kyoto-shi Kyoto 602-8585 (JP); YOSHIMOTO Syuhei, Kyoto-shi Kyoto 602-8585 (JP); TORAI Shinji, Kyoto-shi Kyoto 602-8585 (JP)
(74) Representative: Goddar, Heinz J.
(86) International application number: PCT/JP2017/018844
(87) International publication number: WO 2017/200089

(57) **Abstract**

A perfusion device (1) and a perfusion method can allocate two routes of perfusate inflow pathways (30) and two routes of perfusate outflow pathways (40) for a liver graft (9). The perfusate inflow pathways (30) have perfusate inflow cannulas (32) connected respectively to the portal vein and the hepatic artery. The perfusate outflow pathways (40) have perfusate outflow cannulas (42) connected respectively to the suprahepatic inferior vena cava and the infrahepatic inferior vena cava. Perfusate is allowed to enter the liver (9) from the perfusate inflow cannulas (32), and the perfusate in the liver (9) is allowed to drain off from the perfusate outflow cannulas (42). This significantly shortens the time length of the liver graft being in an ischemic condition when the liver is removed or transplanted. That is, the onset of disorder in the liver graft (9) can be suppressed. This increases the success rate of liver transplantation. Thus, deterioration of organ grafts during surgery is prevented in organ transplantation surgery that requires long hours.

## Description

### Technical Field

The present invention relates to a perfusion device for a liver graft and for use in removing a liver graft from a donor or for use in transplanting a liver graft into a recipient. The present invention also relates to a method of removing a liver graft from a donor and a method of transplanting a liver graft into a recipient.

### Background Art

Organ transplantation is currently performed as the main therapy for irreversible organ dysfunction due to illnesses or accidents. Although the number of transplantation cases has increased and the success rates thereof have dynamically risen with advances in immunosuppressing agents or transplantation technologies, chronic organ shortages are posing a serious problem in transplantation medical care. Even though a method for transplanting a transplant animal organ, a development of gene-modified animals with less tendency to cause immunological rejection, and further a development of artificial organs aiming to replace organ functions with artificial materials have been promoted in order to accommodate for this organ shortage, none of the technology developments have reached a point of replacing a living-donor organ function.

The shortage of donor organs provided for transplantation is not only because of the number of organs provided, but the short duration that the removed organ can be preserved in a transplantable state is also one great reason. For this reason, development of technology to preserve the removed organ ex vivo for a long time in a transplantable state has been promoted. The method currently most broadly employed is a simple cooling method of replacing the blood in the organ with a low-temperature organ preservation solution and then immersing in a low-temperature preservation solution to suppress cell metabolism. There is also a perfusion cooling preservation method that immerses and preserves an organ at a low temperate while perfusing the vascular plexus in the organ with a low-temperature organ preservation solution in order to eliminate waste products in the organ in preservation, which is recently under trial in Europe and the U.S. (for example, Non-Patent Literature 1). However, safe expiration time of organs preserved by these methods is thought to be 60 hours for kidneys and 20 hours for livers in general, and an elongation technique for further duration of preservation has been desired.

Moreover, in addition to the above problems, another factor causing the shortage in the number of donor organs is that organs that can be provided are limited because the majority of donor registrants die of cardiac arrest. In organ transplantation from cardiac arrest donors, in contrast to organ transplantation from brain-dead donors, a period during which the bloodstream to organs is stopped, in other words a period of "warm ischemia," occurs from cardiac arrest until removal and preservation of organs. Cell swelling disorder due to depletion of ATP or accumulation of waste products such as hypoxanthine is caused in an organ or tissue in a warm ischemic state. The hypoxanthine accumulated in cells is rapidly metabolized by the oxygenated perfusate when bloodstream to the organ or tissue is resumed. During this process, tissue disorder may be provoked by the large amount of reactive oxygen produced, and systemic acute shock may be evoked in the recipient receiving the organ transplantation by cytokines etc. secreted from cells.

A method described in Patent Literature 1, for example, is proposed as a long-term preservation method for organs or tissues, for use in transplanting an organ or tissue that is in a warm ischemic state.

### Prior Art Documents

### Patent Literature

Patent Literature 1: International Patent Publication No. WO 2014/038473

### Non-Patent Literature

Non-Patent Literature 1: Moers C. et al., N. Engl. J. Med. 360(1):7, 2009

### Summary of Invention

### Problems to be Solved by Invention

In general, surgery takes long hours for organ removal and organ transplantation (5 to 10 hours for heart transplantation, approximately 3 to 4 hours for renal transplantation, and approximately 8 to 15 hours for liver transplantation). During the surgery of removal and transplantation of an organ, an organ graft gets into an ischemic state and deteriorates. For this reason, a technique to prevent organ graft deterioration or a technique to resuscitate an organ graft damaged by ischemia has been sought. Especially in the liver transplantation, techniques such as described above are more strongly desired because, even though the time is short for liver grafts to be preserved, transplantation surgery requires long hours.

In view of this, it is an object of the present invention to provide a technique for preventing deterioration of organ grafts during surgery and prolonging the preservation time length for organ grafts for organ removal surgery and organ transplantation surgery that require long hours. A further object of the present invention is to provide a technique for resuscitating an organ graft that is damaged by being in an ischemic state.

### Means for Solving Problems

The inventors of the present invention have focused on the vascular structure specific to the liver and found that the ischemic state of the liver during liver transplantation surgery can be prevented by using a perfusion method in which perfusate is allowed to enter the portal vein and the hepatic artery and perfusate is allowed to drain off from the suprahepatic inferior vena cava and the infrahepatic inferior vena cava (i.e., a perfusion method that allows perfusate to enter from two routes of blood vessels and allows perfusate to drain off from two routes of blood vessels), and thus achieved the completion of the present invention.

A first aspect of the present invention is a perfusion device for a liver graft and for use in removing a liver graft from a donor or for use in transplanting a liver graft into a recipient. The device includes the following (A) to (D): (A) the following cannulas (i) to (iv) that includes (i) a perfusate inflow cannula connected to a portal vein of the liver graft, (ii) a perfusate inflow cannula connected to a hepatic artery of the liver graft, (iii) a perfusate outflow cannula connected to a suprahepatic inferior vena cava of the liver graft, and (iv) a perfusate outflow cannula connected to an infrahepatic inferior vena cava of the liver graft, (B) one or a plurality of perfusate containers containing perfusate, (C) one or a plurality of pumps, and (D) a pipe. Here, the (A) to (D) are configured such that, when the liver graft is perfused, at least one of the one or a plurality of pumps allows the perfusate contained in at least one of the one or a plurality of perfusate containers to enter the liver graft from the perfusate inflow cannula (i) and/or the perfusate inflow cannula (ii) through the pipe, and allows the perfusate in the liver graft to drain off from the perfusate outflow cannula (iii) and/or the perfusate outflow cannula (iv).

A second aspect of the present invention is the perfusion device according to the first aspect and characterized in that, when the liver graft is perfused, the perfusate inflow cannula (i) and/or the perfusate inflow cannula (ii), the liver graft, the perfusate outflow cannula (iii) and/or the perfusate outflow cannula (iv), and the perfusate containers form a perfusion circuit through the pipe.

A third aspect of the present invention is the perfusion device according to the first or second aspect and characterized in that, when the device is used to remove a liver graft from a donor, the device is used in a method including the following steps (1) to (4) of: (1) incising or sectioning one of the hepatic artery and the portal vein of the donor to connect first one of the perfusate inflow cannulas to an incised or sectioned area, and incising or sectioning one of the suprahepatic inferior vena cava and the infrahepatic inferior vena cava of the donor to connect first one of the perfusate outflow cannulas to an incised or sectioned area, (2) allowing perfusate to enter from the first perfusate inflow cannula and allowing perfusate to drain off from the first one of the perfusate outflow cannulas to start perfusion of the liver graft with perfusate, (3) incising or sectioning the other of the hepatic artery and the portal vein, the other having not been incised or sectioned in step (1), to connect second one of the perfusate inflow cannulas to an incised or sectioned area, and incising or sectioning the other of the suprahepatic inferior vena cava and the infrahepatic inferior vena cava, the other having not been incised or sectioned in step (1), to connect second one of the perfusate outflow cannulas to an incised or sectioned area, and (4) allowing perfusate to enter from the first and second ones of the perfusate inflow cannulas and allowing perfusate to drain off from the first and second ones of the perfusate outflow cannulas to remove a liver from the donor while maintaining the perfusion of the liver graft with perfusate.

A fourth aspect of the present invention is the perfusion device according to the first or second aspect and characterized in that, when the device is used to transplant a liver graft into a recipient, the device is used in a method including the following steps (5) to (7) of: (5) preparing a liver graft, with the perfusate inflow cannula (i) connected to the portal vein, the perfusate inflow cannula (ii) connected to the hepatic artery, the perfusate outflow cannula (iii) connected to the suprahepatic inferior vena cava, and the perfusate outflow cannula (iv) connected to the infrahepatic inferior vena cava, and with perfusate allowed to enter from the perfusate inflow cannulas (i) and (ii) and perfusate allowed to drain off from the perfusate outflow cannulas (iii) and (iv), (6) extracting the perfusate inflow cannula from one of the portal vein and the hepatic artery of the liver graft and extracting the perfusate outflow cannula from one of the suprahepatic inferior vena cava and the infrahepatic inferior vena cava of the liver graft, and while maintaining the perfusion of the liver graft with perfusate, anastomosing the blood vessel from which the perfusate inflow cannula has been extracted to a corresponding blood vessel of the recipient and anastomosing the blood vessel from which the perfusate outflow cannula has been extracted to a corresponding blood vessel of the recipient, and (7) extracting unextracted one of said perfusate inflow cannulas from the other of the portal vein and the hepatic artery of the liver graft, extracting unextracted one of said perfusate outflow cannulas from the other of the suprahepatic inferior vena cava and the infrahepatic inferior vena cava of the liver graft, anastomosing the blood vessel from which the perfusate inflow cannula has been extracted to a corresponding blood vessel of the recipient, and anastomosing the blood vessel from which the perfusate outflow cannula has been extracted to a corresponding blood vessel of the recipient.

A fifth aspect of the present invention is the perfusion device according to any one of the first to fourth aspects and further includes a container capable of immersing at least part of the liver graft in liquid.

A sixth aspect of the present invention is the perfusion device according to any one of the first to fifth aspects and characterized in that at least part of the pipe is made of an elastic material or has an elastic structure.

A seventh aspect of the present invention is the perfusion device according to the sixth aspect and characterized in that the elastic structure is a bellows structure.

An eighth aspect of the present invention is the perfusion device according to any one of the first to seventh aspects and characterized in that the pipe is provided with a flowmeter that measures a flow rate of perfusate and a manometer that measures a flow pressure of perfusate, the pump has an operation control system, and when the liver graft is perfused, the operation control system of the pump operates in accordance with a measured value of the flowmeter and/or a measured value of the manometer to maintain the flow rate and/or flow pressure of perfusate during perfusion within a predetermined range.

A ninth aspect of the present invention is the perfusion device according to any one of the first to eighth aspects and characterized in that a degassing unit that eliminates bubbles in perfusate is provided in the pipe that connects the perfusate containers and the perfusate inflow cannula (i) or (ii).

A tenth aspect of the present invention is the perfusion device according to any one of the first to ninth aspects and characterized in that the perfusate containers include a temperature control system that regulates a temperature of perfusate and/or a gas exchange system that regulates a volume of dissolved oxygen, dissolved carbon dioxide and/or dissolved nitrogen in perfusate.

An eleventh aspect of the present invention is the perfusion device according to any one of the first to tenth aspects and characterized in that a temperature control unit that regulates a temperature of perfusate is provided in the pipe that connects the perfusate containers and the perfusate inflow cannula (i) or (ii).

A twelfth aspect of the present invention is the perfusion device according to the tenth or eleventh aspect and characterized in that, when the liver graft is perfused, the temperature of the perfusate that enters the liver graft is controlled in a range of 20 to 25°C by the temperature control system of the perfusate containers and/or the temperature control unit provided in the pipe.

A thirteenth aspect of the present invention is the perfusion device according to any one of the first to twelfth aspects and characterized in that, when the liver graft is perfused, the perfusate contains an oxygen carrier.

A fourteenth aspect of the present invention is the perfusion device according to the thirteenth aspect and characterized in that the oxygen carrier is an erythrocyte.

A fifteenth aspect of the present invention is a method of removing a liver graft from a donor, including the steps of (1) incising or sectioning one of a hepatic artery and a portal vein of a donor to connect a first perfusate inflow cannula to an incised or sectioned area, and incising or sectioning one of a suprahepatic inferior vena cava and a infrahepatic inferior vena cava of the donor to connect a first perfusate outflow cannula to an incised or sectioned area, (2) allowing perfusate to enter from the first perfusate inflow cannula and allowing perfusate to drain off from the first perfusate outflow cannula to start perfusion of the liver graft with perfusate, (3) incising or sectioning the other of the hepatic artery and the portal vein, the other having not been incised or sectioned in step (1), to connect a second perfusate inflow cannula to an incised or sectioned area, and incising or sectioning the other of the suprahepatic inferior vena cava and the infrahepatic inferior vena cava, the other having not been incised or sectioned in step (1), to connect a second perfusate outflow cannula to an incised or sectioned area, and (4) allowing perfusate to enter from the first and second perfusate inflow cannulas and allowing perfusate to drain off from the first and second perfusate outflow cannulas to remove a liver from the donor while maintaining the perfusion of the liver graft with perfusate.

A sixteenth aspect of the present invention is the method according to the fifteenth aspect and characterized in that the steps (1) to (4) are performed while the liver of the donor is placed in a range of 20 to 25°C.

A seventeenth aspect of the present invention is the method according to the fifteenth or sixteenth aspect and characterized in that the perfusate used in the steps (1) to (4) contains an oxygen carrier.

An eighteenth aspect of the present invention is the method according to the seventeenth aspect and characterized in that the oxygen carrier is an erythrocyte.

A nineteenth aspect of the present invention is a method of transplanting a liver graft into a recipient, including the steps of (5) preparing a liver graft, with first one of perfusate inflow cannulas connected to a portal vein, second one of perfusate inflow cannulas connected to a hepatic artery, first one of perfusate outflow cannulas connected to a suprahepatic inferior vena cava, and second one of perfusate outflow cannulas connected to an infrahepatic inferior vena cava, and with perfusate allowed to enter the first and second ones of perfusate inflow cannulas and perfusate allowed to drain off from the first and second ones of perfusate outflow cannulas, and (6) extracting a perfusate inflow cannula from one of the portal vein and the hepatic artery of the liver graft and extracting a perfusate outflow cannula from one of the suprahepatic inferior vena cava and the infrahepatic inferior vena cava of the liver graft, and while maintaining the perfusion of the liver graft with perfusate, anastomosing the blood vessel from which the perfusate inflow cannula has been extracted to a corresponding blood vessel of the recipient and anastomosing the blood vessel from which the perfusate outflow cannula has been extracted to a corresponding blood vessel of the recipient.

A twentieth aspect of the present invention is the method according to the nineteenth aspect and further includes the step of (7) extracting unextracted one of the perfusate inflow cannulas from the other of the portal vein and the hepatic artery of the liver graft, extracting unextracted one of the perfusate outflow cannulas from the other of the suprahepatic inferior vena cava and the infrahepatic inferior vena cava of the liver graft, anastomosing the blood vessel from which the perfusate inflow cannula has been extracted to a corresponding blood vessel of the recipient, and anastomosing the blood vessel from which the perfusate outflow cannula has been extracted to a corresponding blood vessel of the recipient.

A twenty-first aspect of the present invention is the method according to the nineteenth aspect and further includes the step of (8) extracting unextracted one of the perfusate inflow cannulas from the other of the portal vein and the hepatic artery of the liver graft, extracting unextracted one of the perfusate outflow cannulas from the other of the suprahepatic inferior vena cava and the infrahepatic inferior vena cava of the liver graft, and ligating the blood vessel from which the perfusate inflow cannula has been extracted or anastomosing the said blood vessel to a corresponding blood vessel of the recipient, and ligating the blood vessel from which the perfusate outflow cannula has been extracted or anastomosing the said blood vessel to a corresponding blood vessel of the recipient.

A twenty-second aspect of the present invention is the method according to any one of the nineteenth to twenty-first aspects and characterized in that each step is performed while the liver graft is placed in a range of 20 to 25°C.

A twenty-third aspect of the present invention is the method according to any one of the nineteenth to twenty-second aspects and characterized in that the perfusate used in each step contains an oxygen carrier.

A twenty-fourth aspect of the present invention is the method according to the twenty-third aspect and characterized in that the oxygen carrier is an erythrocyte.

Note that the scope of the present invention also includes an invention for which one or more features of the present invention listed above are freely combined.

### Effects of the Invention

According to the first to fourteenth aspects of the invention of the present application, two routes of perfusate inflow pathways and two routes of perfusate outflow pathways can be allocated for the liver graft. This significantly shortens the time length of the liver graft 9 being in an ischemic state when the liver graft 9 is removed from a donor or when the liver graft is transplanted into a recipient. In other words, the onset of disorder in the liver graft 9 can be suppressed. Therefore, the success rate of liver transplantation can be improved.

In addition, a current of perfusate when perfusion is performed with the two routes of perfusate inflow pathways and the two routes of perfusate outflow pathways is closer to the condition of bloodstream in vivo than when perfusion is performed with one route of perfusate inflow pathway and one route of perfusate outflow pathway. This suppresses the onset of disorder in the liver during perfusion and preservation after removal from a living body. Furthermore, the function of the liver can recover during perfusion and preservation.

According to the fifteenth to eighteenth aspects of the invention of the present application, in step (1), one of the hepatic artery and the portal vein of the donor and one of the suprahepatic inferior vena cava and the infrahepatic inferior vena cava are connected to the cannulas while the bloodstream is maintained in the other of the hepatic artery and the portal vein of the donor and the other of the suprahepatic inferior vena cava and the infrahepatic inferior vena cava. In this way, the liver 9 to be removed can be protected from being in an ischemic state during the process of connecting one of the hepatic artery and the portal vein of the donor and one of the suprahepatic inferior vena cava and the infrahepatic inferior vena cava to the cannulas. This suppresses the onset of disorder in the liver to be removed.

In addition, in step (3), single flow perfusion is performed with one of the hepatic artery and the portal vein of the donor and one of the suprahepatic inferior vena cava and the infrahepatic inferior vena cava. Therefore, in step (3), the liver 9 to be removed can be protected from being in an ischemic state during the process of connecting the other of the hepatic artery and the portal vein of the donor and the other of the suprahepatic inferior vena cava and the infrahepatic inferior vena cava to the cannulas. This further suppresses the onset of disorder in the liver 9 to be removed.

According to the nineteenth to twenty-fourth aspects of the invention of the present application, in step (6), one of the hepatic artery and the portal vein of the donor and one of the suprahepatic inferior vena cava and the infrahepatic inferior vena cava of the donor are blocked and anastomosed in advance to the blood vessels of the recipient. Even during the process of anastomosing these two blood vessels, the single flow perfusion is maintained in the other of the hepatic artery and the portal vein and the other of the suprahepatic inferior vena cava and the infrahepatic inferior vena cava. In this way, the liver graft 9 can be protected from being in an ischemic state during transplantation surgery. Thus, it is possible to provide ample time to anastomose two blood vessels that are sutured in advance with blood vessels of the recipient.

### BRIEF DESCRIPTION OF THE DRAWINGS

Fig. 1 is a schematic diagram showing a design example of a perfusion device for a liver graft according to one embodiment of the present invention;
Fig. 2 is a flowchart showing the procedure of a process of removing a liver graft from a donor with the perfusion device of the present invention;
Fig. 3 is a flowchart showing the procedure of a process of transplanting a liver graft into a recipient with the perfusion device of the present invention;
Fig. 4 is a flowchart showing another example of the procedure of the process of transplanting a liver graft into a recipient with the perfusion device of the present invention;
Fig. 5 illustrates a liver graft that is being perfused with the device of the present invention after removed from a donor;
Fig. 6 illustrates the liver graft in Fig. 2 being transplanted;
Fig. 7 illustrates the results of experiments conducted to assess a disorder suppressing effect achieved by temperature control when the liver graft is removed and perfused with the device and method of the present invention;
Fig. 8 illustrates the results of experiments conducted to assess a disorder suppressing effect achieved by the supplement of erythrocytes to the perfusate when the liver graft is removed and perfused with the device and method of the present invention;
Fig. 9 illustrates the results of experiments conducted in order to confirm that the liver perfused with the device and method of the present invention can maintain its sufficient function in vivo;
Fig. 10 illustrates the results of experiments showing that the device and method of the present invention are available for a liver donated from a cardiac arrest donor (donation after cardiac death (DCD) donor);
Fig. 11 illustrates the survival rate of recipients with ischemic livers transplanted after perfusion using the device and method of the present invention;

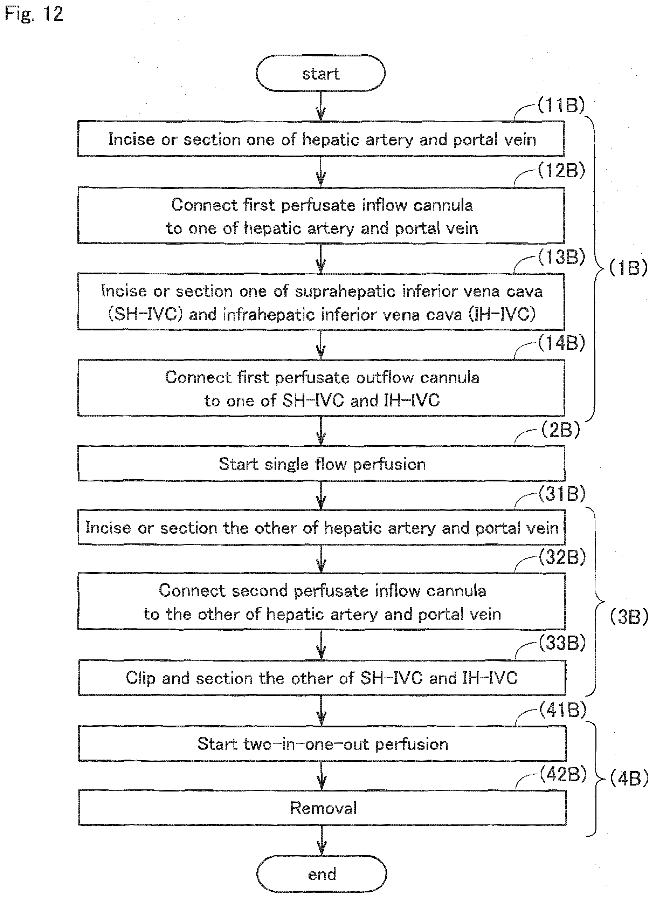

Fig. 12 is a flowchart showing the procedure of a process of removing a liver graft from a donor with a two-in-one-out perfusion; and

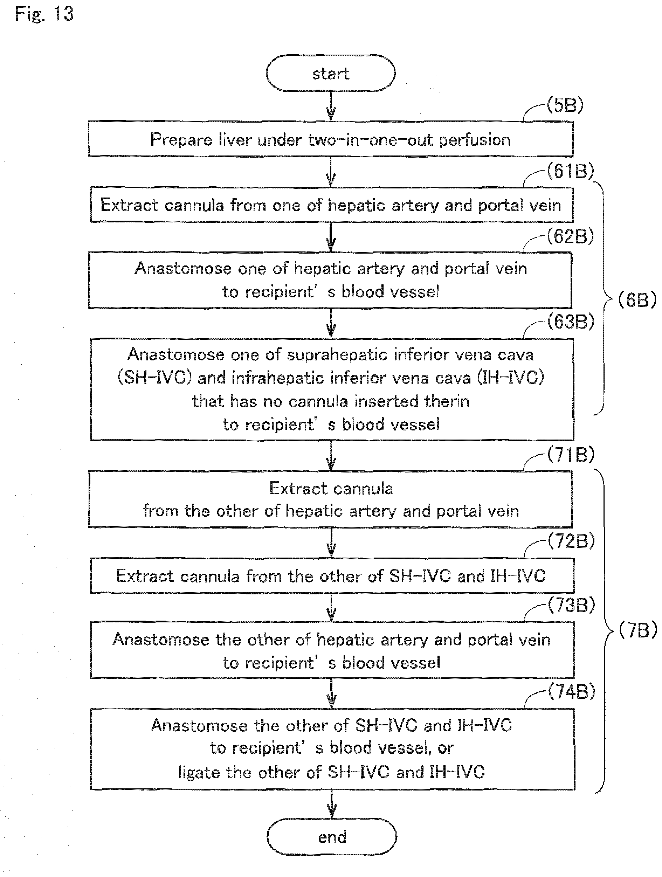

Fig. 13 is a flowchart showing the procedure of a process of transplanting a liver graft into a recipient with a two-in-one-out perfusion.

### DESCRIPTION OF PREFERRED EMBODIMENTS

A design example of a perfusion device, a liver removal method and liver transplantation method using the perfusion device, and examples of liver transplantation experiments according to the present invention are described below. In the present application, donors and recipients may be humans or non-human animals. Also, non-human animals may be rodents such as mice and rats, ungulates such as pigs, goats, and sheep, non-human primates such as chimpanzees, and other non-human mammals, and may be nonmammalian animals.

### <1. Design Example of Perfusion Device>

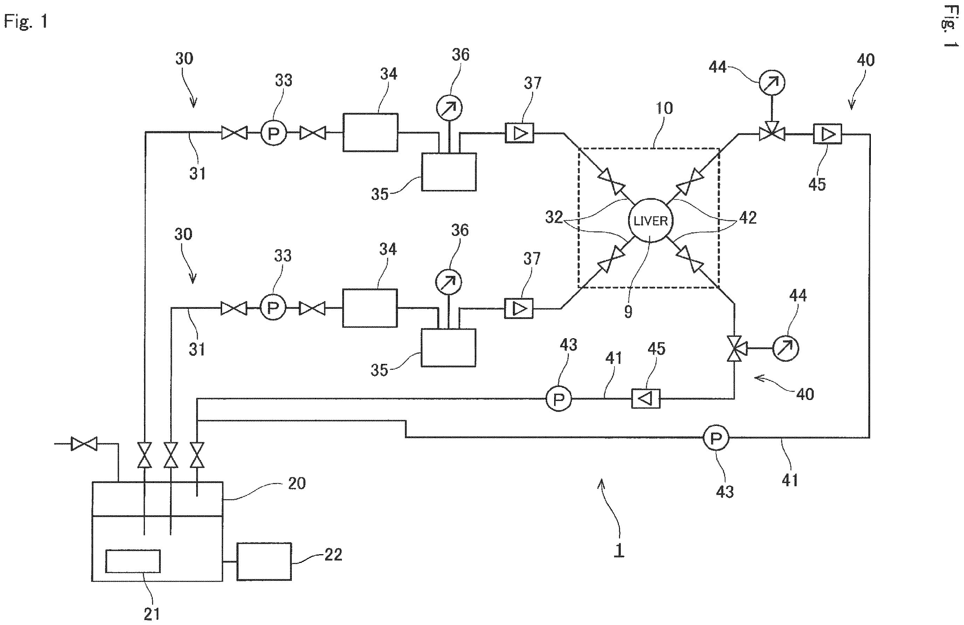

A device according to the present invention is described with reference to Fig. 1 that shows a design example of a perfusion device 1 for a liver graft 9 as one embodiment of the present invention.

The perfusion device 1 in the example of Fig. 1 includes a bioreactor area 10 that stores the liver graft 9, a perfusate reservoir 20, two perfusate inflow pathways 30, and two perfusate outflow pathways 40.

In the bioreactor area 10, a container capable of immersing at least part of the liver graft 9 in a liquid such as perfusate is placed. The perfusate reservoir 20 is a perfusate container that pools perfusate. In addition, the perfusate reservoir 20 includes a temperature control system 21 and a gas exchange system 22.

Each perfusate inflow pathway 30 includes a pipe 31, a perfusate inflow cannula 32, a pump 33 inserted in the pipe 32, a temperature control unit 34, a degassing unit 35, a manometer 36, and a flowmeter 37. The pipe 31 connects the perfusate reservoir 20 and the perfusate inflow cannula 32. The perfusate inflow cannula 32 is connected to a blood vessel of the liver graft 9. The pump 33 creates a current of perfusate inside the pipe 31 from the perfusate reservoir 20 to the perfusate inflow cannula 32.

Each perfusate outflow pathway 40 includes a pipe 41, a perfusate outflow cannula 42, a pump 43 inserted in the pipe 41, a manometer 44, and a flowmeter 45. The pipe 41 connects the perfusate outflow cannula 42 and the perfusate reservoir 20. The perfusate outflow cannula 42 is connected to a blood vessel of the liver graft 9. The pump 43 creates a current of perfusate inside the pipe 41 from the perfusate outflow cannula 42 to the perfusate reservoir 20.

In the example of Fig. 1, the liver graft 9 is stored in the bioreactor area 10. The portal vein and the hepatic artery of the liver graft 9 are connected to each perfusate inflow cannula 32. The suprahepatic inferior vena cava (SH-IVC) and the infrahepatic inferior vena cava (IH-IVC) of the liver 9 are connected to each perfusate outflow cannula 42.

The cannulas 32 and 42 are further connected to the pipes 31 and 41, respectively. With the driving of the pump 33, the perfusate inside the perfusate reservoir 20 (perfusate container) is allowed to enter the liver 9 through the pipe 31 and the perfusate inflow cannula 32. Whereas, with the driving of the pump 43, the perfusate entering the liver 9 is allowed to drain off through the perfusate outflow cannula 42 to the pipe 41 and returns to the perfusate reservoir 20.

As described above, this perfusion device 1 includes the following (A) to (D):
(A) cannulas (i) to (iv) including:
   (i) a perfusate inflow cannula 32 connected to the portal vein of the liver graft 9;
   (ii) a perfusate inflow cannula 32 connected to the hepatic artery of the liver graft 9;
   (iii) a perfusate outflow cannula 42 connected to the suprahepatic inferior vena cava of the liver graft 9;
   (iv) a perfusate outflow cannula 42 connected to the infrahepatic inferior vena cava of the liver graft 9,
(B) one or more perfusate reservoirs 20 (perfusate container) containing perfusate;
(C) one or more pumps 33 and 43; and
(D) pipes 31 and 41.

The device is configured such that, when the liver graft 9 is perfused, at least one of the pumps 33 and 43 allows perfusate contained in at least one of the perfusate reservoirs 20 to enter the liver graft 9 through the pipe 31 or 41 from one or both of the two perfusate inflow cannulas 32 and allows perfusate in the liver graft 9 to drain off from one or both of the perfusate outflow cannulas 42.

Fig. 1 shows an example in which the perfusate inflow cannulas 32, the liver graft 9, the perfusate outflow cannulas 42, and a single perfusate reservoir 20 form a perfusion circuit through the pipes 31 and 41. In other words, in the example of Fig. 1, the perfusate inflow cannula(s) (i) and/or (ii), the liver graft, the perfusate outflow cannula(s) (iii) and/or (iv), and the perfusate reservoir form a perfusion circuit through the pipes when the liver graft 9 is perfused.

However, the device of the present invention does not have to form a perfusion circuit. For example, the device may be configured to dispose of perfusate having passed through the liver 9, or may be configured to drain perfusate to another perfusate reservoir that is different from the perfusate reservoir in which the perfusate has been preserved before passing through the liver 9.

As described above, the perfusion device of the present invention is characterized in that two routes of perfusate inflow pathways 30 and two routes of perfusion outflow pathways 40 are allocated for the liver graft 9. Configuring the perfusion device as above significantly shortens the time length of the liver graft 9 being in an ischemic state when the liver graft 9 is removed from a donor or when the liver graft 9 is transplanted into a recipient. In other words, the onset of disorder in the liver graft 9 can be suppressed. This potentially raises the success rate of transplantation of the liver 9.

In addition, a current of perfusate in perfusion performed with the two routes of perfusate inflow pathways 30 and the two routes of perfusate outflow pathways 40 is closer to the condition of bloodstream in vivo than in perfusion performed with one route of perfusate inflow pathway and one route of perfusate outflow pathway. This suppresses the onset of disorder in the liver 9 during perfusion and preservation after removal from a living body. Furthermore, the function of the liver 9 can recover during perfusion and preservation.

### <2. Liver Transplantation Using Perfusion Device>

Next, a procedure for liver transplantation using the perfusion device 1 of the present invention is described below.

### <2-1. Procedure for Donor Liver Removal Using Perfusion Device>

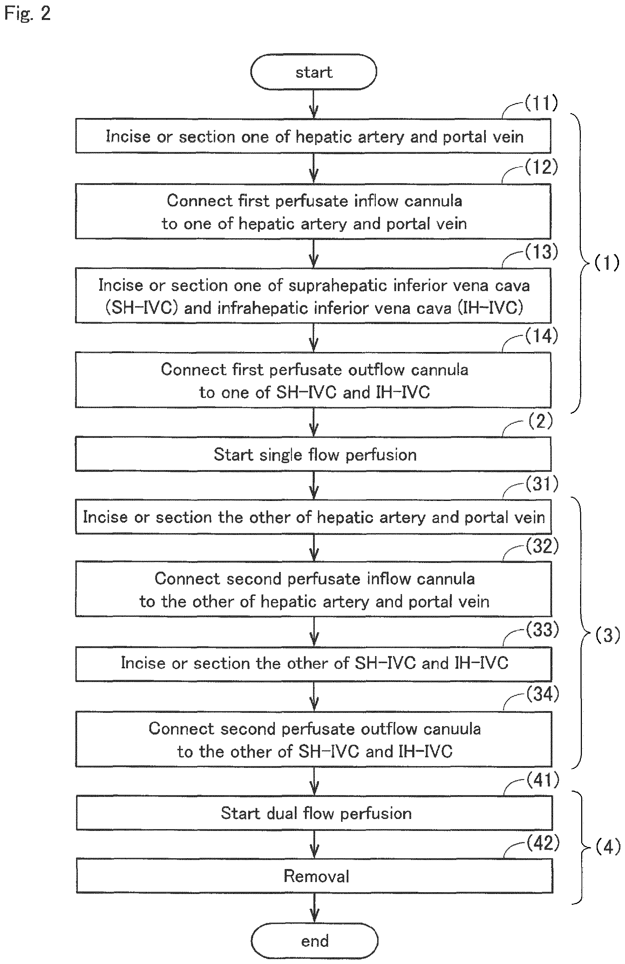

First, a procedure for removing a donor liver using the perfusion device 1 of the present invention is described with reference to Fig. 2. Fig. 2 is a flowchart showing the procedure of a process of removing the liver graft 9 from a donor with the perfusion device 1 of the present invention. For example, the following steps (1) to (4) may be performed when the liver graft 9 is removed from a donor with the perfusion device 1 of the present invention.

In step (1), one of the hepatic artery and the portal vein of the donor is incised or sectioned (step (11)). Then, a first perfusate inflow cannula 32 is connected to the incised or sectioned areas (step (12)). Next, one of the suprahepatic inferior vena cava and the infrahepatic inferior vena cava of the donor is incised or sectioned (step (13)). Then, a first perfusate outflow cannula 42 is connected to the incised or sectioned area (step (14)).

Next, in step (2), perfusate is allowed to enter from the first perfusate inflow cannula 32, and perfusate is allowed to drain off from the first perfusate outflow cannula 42. This starts perfusion of the liver graft 9 with perfusate. Accordingly, single flow perfusion is started with one route of perfusate inflow pathway 30 and one route of perfusate outflow pathway 40.

Subsequently, in step (3), the other of the hepatic artery and the portal vein that has not been incised or sectioned in step (1) is first incised or sectioned (step (31)). Then, a second perfusate inflow cannula 32 is connected to the incised or sectioned area (step (32)). Subsequently, the other of the suprahepatic inferior vena cava and the infrahepatic inferior vena cava that has not been incised or sectioned in step (1) is incised or sectioned (step (33)). Then, a second perfusate outflow cannula 42 is connected to the incised or sectioned area (step (34)).

Thereafter, in step (4), perfusate is allowed to enter from the first and second perfusate inflow cannulas 32, and perfusate is allowed to drain off from the first and second perfusate outflow cannulas 42. In other words, dual-flow perfusion starts (step (41)). Then, the liver 9 is removed from a donor while the perfusion of the liver graft 9 with perfusate is maintained (step (42)).

In step (1) described above, the combination of blood vessels to be incised or sectioned (i.e., the combination of blood vessels that are incised or sectioned in advance) is not limited and can be appropriately selected by those skilled in the art in consideration of conditions such as the volume of the bloodstream of each blood vessel and organ status. Any combinations are allowed, for example, a combination of the hepatic artery and the suprahepatic inferior vena cava, a combination of the hepatic artery and the infrahepatic inferior vena cava, a combination of the portal vein and the suprahepatic inferior vena cava, and a combination of the portal vein and the infrahepatic inferior vena cava.

For each combination, the order of blood vessels to be incised or sectioned is not limited and can be appropriately selected by those skilled in the art in consideration of conditions such as the volume of the bloodstream of each blood vessel and organ status. In step (1), step (12) needs to be performed at least after step (11), and step (14) needs to be performed at least after step (13). Furthermore, in step (3), step (32) needs to be performed at least after step (31), and step (34) needs to be performed at least after step (33).

In steps (1) to (4) described above, treatments normally performed in the procedure for removing the liver graft 9 from a donor (for example, excision of connective tissues, peeling of blood vessels, temporary ligation or clamping of blood vessels for section of blood vessel, blockage and dissection of the bile duct, application of coagulation agents to the liver graft 9, hemostasis for operated sites) can be conducted as needed by those skilled in the art.

In step (1) described above, one of the hepatic artery and the portal vein of the donor and one of the suprahepatic inferior vena cava and the infrahepatic inferior vena cava are connected to the cannulas while the blood flow is maintained in the other of the hepatic artery and the portal vein of the donor and the other of the suprahepatic inferior vena cava and the infrahepatic inferior vena cava. In this way, the liver 9 to be removed can be protected from being in an ischemic state during the process of connecting one of the hepatic artery and the portal vein of the donor and one of the suprahepatic inferior vena cava and the infrahepatic inferior vena cava to the cannulas. This suppresses the onset of disorder in the liver 9 to be removed.

In addition, in step (3) described above, single flow perfusion is performed with one of the hepatic artery and the portal vein of the donor and one of the suprahepatic inferior vena cava and the infrahepatic inferior vena cava. Therefore, in step (3), the liver 9 to be removed can be protected from being in an ischemic state during the process of connecting the other of the hepatic artery and the portal vein of the donor and the other of the suprahepatic inferior vena cava and the infrahepatic inferior vena cava to the cannulas. This further suppresses the onset of disorder in the liver 9 to be removed.

Note that steps (1) to (4) described above are preferably performed in the condition where the liver 9 of the donor is placed in the range of 20 to 25°C as much as possible. For this reason, when the liver 9 is perfused in steps (2) to (4) described above, the temperature of the perfusate entering the liver 9 is preferably controlled in the range of 20 to 25°C by the temperature control system 21 of the perfusate reservoir 20 and/or the temperature control unit 34 provided in the pipe 31.

Furthermore, the perfusate used in steps (1) to (4) described above preferably contains an oxygen carrier. The oxygen carrier is, for example, erythrocyte or artificial erythrocyte.

### <2-2. Procedure for Liver Transplantation into Recipient Using Perfusion Device>

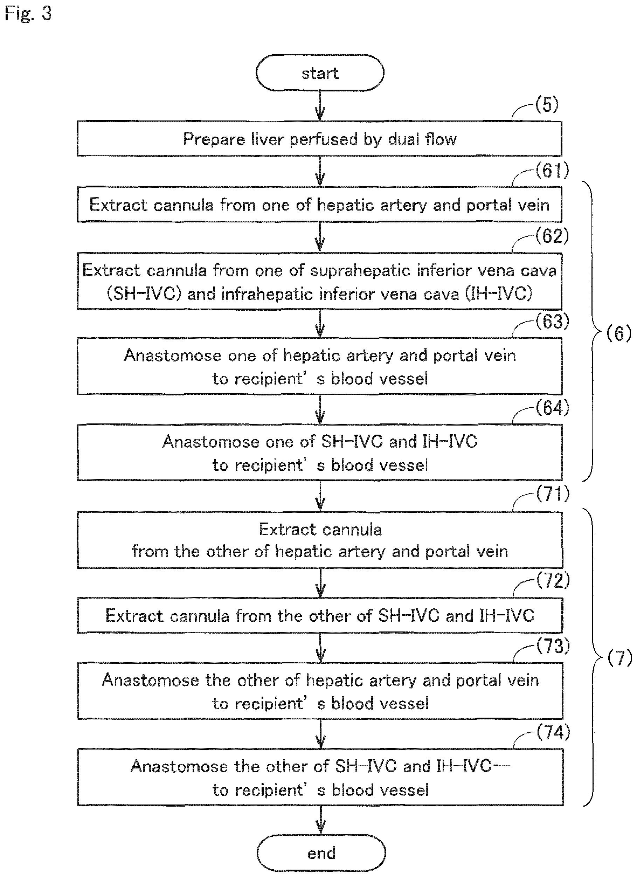

Next, the procedure of transplanting a donor liver into a recipient with the perfusion device 1 of the present invention is described with reference to Fig. 3. Fig. 3 is a flowchart showing the procedure of the process of transplanting the liver graft 9 into a recipient with the perfusion device 1 of the present invention. For example, the following steps (5) to (7) may be performed when the perfusion device 1 of the present invention is used to transplant the liver graft 9 into a recipient.

In the following description, the perfusate inflow cannula 32 connected to the portal vein of the liver 9 is referred to as a perfusate inflow cannula 32(i), the perfusate inflow cannula 32 connected to the hepatic artery of the liver 9 as a perfusate inflow cannula 32(ii), the perfusate outflow cannula 42 connected to the suprahepatic inferior vena cava of the liver 9 as a perfusate outflow cannula 42(iii), and the perfusate outflow cannula 42 connected to the infrahepatic inferior vena cava as a perfusate outflow cannula 42(iv).

First, in step (5), the liver graft 9 is prepared, with the perfusate inflow cannula 32(i) connected to the portal vein, the perfusate inflow cannula 32(ii) connected to the hepatic artery, the perfusate outflow cannula 42(iii) connected to the suprahepatic inferior vena cava, and the perfusate outflow cannula 42(iv) connected to the infrahepatic inferior vena cava, and with perfusate allowed to enter from the perfusate inflow cannulas 32(i) and 32(ii) and perfusate allowed to drain off from the perfusate outflow cannulas 42(iii) and 42(iv). In other words, the liver 9 that has been perfused as a whole is prepared.

Next, in step (6), the perfusate inflow cannulas 32 is extracted from one of the portal vein and the hepatic artery of the liver graft 9 (step (61)). In addition, the perfusate outflow cannulas 42 is extracted from one of the suprahepatic inferior vena cava and the infrahepatic inferior vena cava of the liver graft 9 (step (62)). Then, the blood vessel from which the perfusate inflow cannula 32 has been extracted is anastomosed to a corresponding blood vessel of the recipient while the perfusion of the liver graft 9 with perfusate is maintained (step (63)). Also, the blood vessel from which the perfusate outflow cannula 42 has been extracted is anastomosed to a corresponding blood vessel of the recipient (step (64)). In this way, single flow perfusion is performed with the unextracted perfusate inflow cannula 32 and the unextracted perfusate outflow cannula 42 during the process of step (6). This prevents the liver graft 9 from being in an ischemic state in step (6).

Next, in step (7), an unextracted perfusate inflow cannula 32 is extracted from the other of the portal vein and the hepatic artery of the liver graft 9 (step (71)). Also, an unextracted perfusate outflow cannula 42 is extracted from the other of the suprahepatic inferior vena cava and the infrahepatic inferior vena cava of the liver graft 9 (step (72)). Then, the blood vessel from which the perfusate inflow cannula 32 has been extracted is anastomosed to a corresponding blood vessel of the recipient (step (73)). Also, the blood vessel from which the perfusate outflow cannula 42 has been extracted is anastomosed to a corresponding blood vessel of the recipient (step (74)).

In step (6) described above, the combination of the blood vessels of the donor liver 9 used for anastomosis (i.e., the combination of blood vessels that are anastomosed in advance) is not limited and can be approximately selected by those skilled in the art in consideration of conditions such as the volume of the bloodstream of each blood vessel and organ status. Any combinations are allowed, for example, a combination of the hepatic artery and the suprahepatic inferior vena cava, a combination of the hepatic artery and the infrahepatic inferior vena cava, a combination of the portal vein and the suprahepatic inferior vena cava, and a combination of the portal vein and the infrahepatic inferior vena cava. For each combination, the order of blood vessels to be anastomosed is not limited and can be appropriately selected by those skilled in the art in consideration of conditions such as the volume of the bloodstream of each blood vessel and organ status.

For this reason, the definition of "one" and "the other" in steps (1) to (4) may be the same as or different from the definition of "one" and "the other" in steps (5) to (7). In other words, the blood vessels from which the cannulas 32 and 42 are extracted in step (6) may be the blood vessels to which the cannulas 32 and 42 are connected in step (1), or may be the blood vessels to which the cannulas 32 and 42 are connected in step (2).

In step (6) described above, the recipient's blood vessels anastomosed to the blood vessels of the donor liver 9 may be the same type of blood vessels as the donor's blood vessels used for anastomosis, or may be a different type of blood vessels. For example, in the orthotopic transplantation of the liver 9, the recipient's blood vessels that are anastomosed to the blood vessels of the donor liver 9 may be the same type of blood vessels as the blood vessels of the donor liver 9 used for anastomosis. Furthermore, for example, in the heterotopic transplantation of the liver 9, the recipient's blood vessels that are anastomosed to the blood vessels of the donor liver 9 may be a different type of blood vessels from the blood vessels of the donor liver 9 used for anastomosis. The type of recipient's blood vessels that are used in the heterotopic transplantation of the liver 9 can be appropriately selected based on common general technical knowledge by those skilled in the art.

In steps (5) to (7) described above, treatments normally performed in the procedure for transplanting the liver graft 9 into a recipient (for example, excision of connective tissues, peeling of blood vessels, temporary ligation or clamping of blood vessels for section and anastomose of the vessels, anastomosis of blood vessels, anastomosis of the bile duct, hemostasis for operated sites) can be conducted as needed by those skilled in the art.

In step (6) described above, one of the hepatic artery and the portal vein of the donor and one of the suprahepatic inferior vena cava and the infrahepatic inferior vena cava of the donor are blocked and anastomosed in advance to the recipient's blood vessels. Even during the process of anastomosing these two blood vessels, single flow perfusion is maintained in the other of the hepatic artery and the portal vein and the other of the suprahepatic inferior vena cava and the infrahepatic inferior vena cava. In this way, the liver graft 9 can be protected from being in an ischemic state during transplantation surgery. Thus, it is possible to provide ample time to anastomose two blood vessels that are sutured in advance with the recipient's blood vessels.

Note that steps (5) to (7) described above are preferably performed in the condition where the liver graft 9 of the donor is placed in the range of 20 to 25°C as much as possible. For this reason, when the liver 9 is perfused in steps (5) to (6) described above, the temperature of the perfusate entering the liver 9 is preferably controlled in the range of 20 to 25°C by the temperature control system 21 of the perfusate reservoir 20 and/or the temperature control unit 34 provided in the pipe 31.

Furthermore, the perfusate used in steps (5) to (7) described above preferably contains an oxygen carrier. The oxygen carrier is, for example, erythrocyte or artificial erythrocyte.

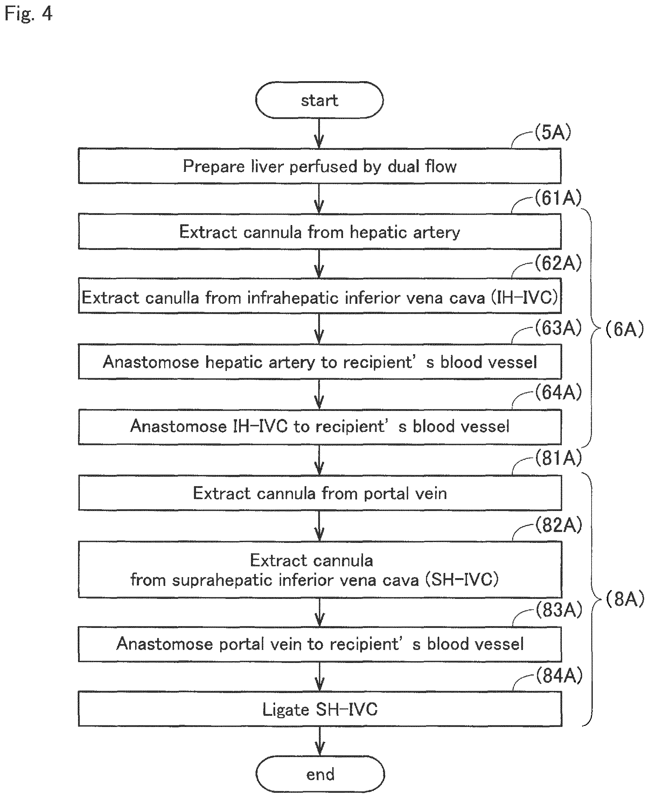

Note that in step (7) described above, all the four blood vessels in which the cannulas 32 and 42 have been inserted are anastomosed to the recipient's blood vessels. However, the present invention is not limited to this. One or two of the four blood vessels may be ligated without being anastomosed to the recipient's blood vessel(s). Fig. 4 is a flowchart showing another example of the process of transplanting the liver graft 9 into a recipient with the perfusion device 1 of the present invention.

First, similarly in step (5) described above, the liver 9 that has been perfused as a whole is prepared in step (5A). In other words, the liver graft 9 is prepared, with the perfusate inflow cannula 32(i) connected to the portal vein, the perfusate inflow cannula 32(ii) connected to the hepatic artery, the perfusate outflow cannula 42(iii) connected to the suprahepatic inferior vena cava, and the perfusate outflow cannula 42(iv) connected to the infrahepatic inferior vena cava, and with perfusate allowed to enter the perfusate inflow cannulas 32(i) and 32(ii) and perfusate allowed to drain off from the perfusate outflow cannulas 42(iii) and 42(iv).

Next, in step (6A), the perfusate inflow cannula 32 is extracted from the hepatic artery of the liver graft 9 (step (61A)). Also, the perfusate outflow cannula 42 is extracted from the infrahepatic inferior vena cava of the liver graft 9 (step (62A)). Then, the hepatic artery from which the perfusate inflow cannula 32 has been extracted is anastomosed to a corresponding blood vessel of the recipient while the perfusion of the liver graft 9 with perfusate is maintained (step (63A)). Also, the infrahepatic inferior vena cava from which the perfusate outflow cannula 42 has been extracted is anastomosed to a corresponding blood vessel of the recipient (step (64A)). In this way, single flow perfusion is performed with the unextracted perfusate inflow cannula 32 and the unextracted perfusate outflow cannula 42 during the process of step (6A). This prevents the liver graft 9 from being in an ischemic state in step (6A).

Next, in step (8A), the perfusate inflow cannula 32 is extracted from the portal vein of the liver graft 9 (step (81A)). Also, the perfusate outflow cannula 42 is extracted from the suprahepatic inferior vena cava of the liver graft 9 (step (82A)). Then, the portal vein from which the perfusate inflow cannula 32 has been extracted is anastomosed to a corresponding blood vessel of the recipient (step (83A)). Also, the suprahepatic inferior vena cava from which the perfusate outflow cannula 42 has been extracted is ligated (step (84A)).

In this way, some of the four blood vessels having been inserted in the cannulas 32 and 42 may be ligated without being anastomosed to the recipient's blood vessels(s). Such a method is, in particular, employed in the case of heterotopic transplantation in which part or whole of the recipient's organ is conserved.

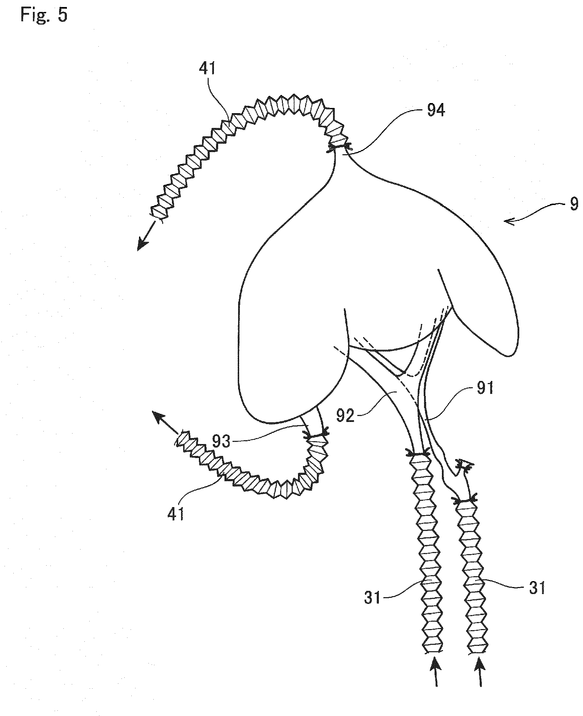

Fig. 5 illustrates the liver graft 9 being perfused with the perfusion device 1 of the present invention after removed from the donor. The hepatic artery 91 and the portal vein 92 are connected to the bellows-structured pipes 31 via the perfusate inflow cannulas 32, and the suprahepatic inferior vena cava 93 and the infrahepatic inferior vena cava 94 are connected to the bellows-structured pipes 41 via the perfusate outflow cannulas 42. Then, perfusate is allowed to enter the liver 9 from the hepatic artery 91 and the portal vein 92, and perfusate is allowed to drain off from the suprahepatic inferior vena cava 93 and the infrahepatic inferior vena cava 94.

In the example of the Fig. 5, at least part of the pipes 31 and 41 has a bellows structure and thereby has elasticity. In this way, at least part of the pipes 31 and 41 is preferably made of an elastic material or has an elastic structure. This makes it easier to change the position or direction of the liver 9 during removal or transplantation surgery of the liver graft 9.

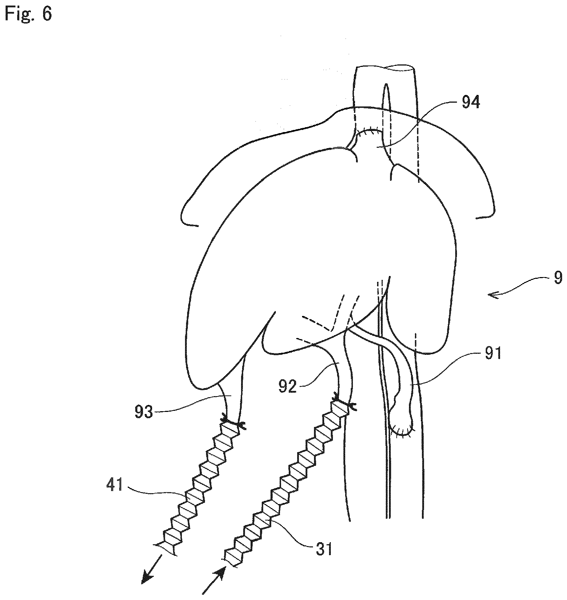

Fig. 6 illustrates the liver graft 9 in Fig. 5 being transplanted. In the illustration of Fig. 6, first, the hepatic artery 91 and the infrahepatic inferior vena cava 94 of the liver graft 9 are blocked and anastomosed in advance to the recipient's blood vessels. Even during the process of anastomosing these two blood vessels, namely, the hepatic artery 91 and the suprahepatic inferior vena cava 93, the perfusion of the liver 9 using the portal vein 92 and the infrahepatic inferior vena cava 94 of the liver graft 9 is maintained. This prevents the liver graft 9 from being in an ischemia state during transplantation surgery. Thus, it is possible to provide ample time to anastomose the two blood vessels, namely, the hepatic artery 91 and the suprahepatic inferior vena cava 93.

### <3. Design Variations of Perfusion Device>

The perfusion device 1 of the present invention may include a container (i.e., a container for a bioreactor) in which at least part of the liver graft 9 can be immersed in a liquid. Immersing the liver graft 9 in a liquid can prevent the liver graft 9 from being dried and allows the liver graft to be maintained at the optimal temperature by controlling the temperature of the liquid used for immersion. The liquid in which the liver graft is immersed may, for example, be a liquid having the same composition as that of the perfusate, and may be physiological saline. Moreover, in the specification of the present application, examples of the "liquid" for immersing the liver 9 include fluidic, gelatinous or solated, and agar-like substances.

The shape, structure, size and materials of the cannulas 32 and 42 used in the present invention are not limited and can be appropriately selected depending on the type of blood vessel by those skilled in the art.

At least part of the pipes 31 and 41 used in the perfusion device 1 of the present invention is preferably made of an elastic material or has an elastic structure. Employing the elastic material or structure for the pipes 31 and 41 can contribute to smooth transfer of the liver graft 9 removed from the living donor's body to the bioreactor area 10 while maintaining the perfused condition of the liver graft. The liver graft 9 can also be transferred smoothly from the bioreactor area 10 to a living recipient's body (or a working table for transplant preparation) while maintaining the perfused condition of the liver 9 in the same manner. Examples of the elastic material that can be used for the pipes 31 and 41 of the present invention include silicon, urethane, elastomer, and fluorocarbon polymer. Also, examples of the elastic structure that can be used for the pipes 31 and 41 of the present invention include a bellows structure and a reel structure.

The structure of the pumps 33 and 43 used in the perfusion device 1 of the present invention is not limited as long as the drive of the pumps can occur transfer of the perfusate in the pipes 31 and 41, and those skilled in the art can appropriately use pumps with a well-known structure on the basis of common general technical knowledge. The pumps used in the perfusion device 1 of the present invention may be provided in only the pipes 31 connected to the perfusate inflow cannulas 32, or may be provided in only the pipes 41 connected to the perfusate outflow cannulas 42, or may be provided in both of the pipes 31 and 41.

The pumps 33 and 43 used in the perfusion device 1 of the present invention may be configured such that their drive is automatically controlled in response to changes in the flow rate and/or flow pressure of perfusate in the pipes 31 and 41. For example, as illustrated in Fig. 1, the flowmeters 37 and 45 for measuring the flow rate of perfusate and the manometers 36 and 44 for measuring the flow pressure of perfusate may be provided in the pipes 31 and 41 of the device. The pumps 33 and 43 may include an operation control system and may be configured to keep the flow rate and/or flow pressure of perfusate during perfusion within a predetermined range by operating the operation control system of the pumps 33 and 43 in response to the measured values of the flowmeters 37 and 45 and/or manometers 36 and 44.

The flow rate and/or flow pressure of the perfusate that enters or drains off from each blood vessel of the liver graft 9 is preferably set and maintained in a different range of appropriate values (for example, between the upper and lower limits of the flow rate and/or flow pressure of blood that flows through the blood vessel in vivo). This automatic control of the flow rate and/or flow pressure of the perfusate flowing through the pipes prevents abrupt changes in the flow rate and/or flow pressure of the perfusate during removal or transplantation of the liver 9, and avoids damage to the liver graft 9.

In the perfusion device 1 of the present invention, a degassing unit 35 for eliminating bubbles in perfusate may be provided in the pipes 31 that connect the perfusate reservoir 20 (perfusate container) and the perfusate inflow cannulas 32 as illustrated in Fig. 1. The elimination of bubbles from the perfusate entering the liver graft 9 can prevent blockage of blood vessels caused by bubbles in the liver graft 9.

In the perfusion device 1 of the present invention, as illustrated in Fig. 1, the perfusate reservoir 20 (perfusate container) may include the temperature control system 21 for regulating the temperature of the perfusate and/or the gas exchange system 22 for regulating the volume of dissolved oxygen, dissolved carbon dioxide, and/or dissolved nitrogen in perfusate. This can maintain the optimal condition of the perfusate entering the liver graft 9.

In the perfusion device 1 of the present invention, as illustrated in Fig. 1, the temperature control unit 34 for regulating the temperature of perfusate may be provided in the pipes 31 that connect the perfusate reservoir 20 (perfusate container) and the perfusate inflow cannulas 32. This can maintain the more optimal condition of the perfusate entering the liver graft 9.

As shown in the embodiment of the present application, the liver graft 9 is preferably preserved in the range of 20 to 25°C. Thus, in the perfusion device of the present invention, the temperature of the perfusate entering the liver graft 9 is preferably controlled in the range of 20 to 25°C by the temperature control system 21 of the above-described perfusate reservoir 20 (perfusate container) and/or the temperature control units 34 provided in the pipes. Furthermore, the temperature of the liver graft 9 can be maintained in the range of 20 to 25°C by controlling the temperature of the liquid in which the liver graft 9 is immersed (i.e., the liquid inside the container of the bioreactor area 10).

The composition of the perfusate used in the perfusion device of the present invention is not limited as long as it is commonly used in the perfusion of a liver graft. Commercially available perfusate (e.g., L-15 medium) may be used as the perfusate used in the perfusion device of the present invention. The perfusate used in the perfusion device of the present invention is preferably supplemented with an oxygen carrier. Containing an oxygen carrier in the perfusate can suppress disorders of the liver graft and can increase the success rate of liver transplantation. Examples of the oxygen carrier used in the present invention include an erythrocyte and an artificial erythrocyte. Erythrocytes supplemented to the perfusate of the present invention are preferably erythrocytes of the blood type available for blood transfusion for the donor or the recipient, and more preferably erythrocytes derived from the donor or the recipient. Also, artificial erythrocytes supplemented to the perfusate of the present invention need only be molecules having a function of transporting oxygen, and examples thereof include perfluorocarbon and hemoglobin vesicles.

According to the present invention, the concentration of oxygen carriers in perfusate can be appropriately set depending on the type of oxygen carriers to be used by those skilled in the art. For example, when erythrocytes are used as oxygen carriers, the erythrocyte concentration in perfusate is preferably in the range of 0.5 × 10¹¹ cells to 50.0 × 10¹¹ cells per liter of the perfusate, more preferably in the range of 1.0 × 10¹¹ cells to 50.0 × 10¹¹ cells per liter of the perfusate, and most preferably in the range of 2.0 × 10¹¹ cells to 50.0 × 10¹¹ cells per liter of the perfusate. If the erythrocyte concentration in perfusate is less than 0.5 × 10¹¹ cells per liter of the perfusate, the supply of oxygen to organs will be insufficient and necrosis of cells in the organ will occur, and if the erythrocyte concentration in perfusate is greater than 50.0 × 10¹¹ cells per liter of the perfusate, organ disorder due to erythrocyte infarction may occur during perfusion.

In the present invention, the "liver graft 9" is not limited to the liver 9 removed from a donor. For example, the liver graft 9 may be an artificial liver derived from a stem cell such as an iPS cell.

The terms as used in the specification of the present invention, unless otherwise specified in detail, are used to describe specific embodiments and do not intend to limit the invention.

Also, the term "including" as used in the specification of the present invention, unless otherwise clearly required by the content, intends to mean the presence of described items (such as components, steps, elements, and numbers), and does not intend to exclude the presence of other items (such as components, steps, elements, and numbers).

Unless otherwise defined, all the terms as used herein (including technical and scientific terms) have the same meanings as those broadly recognized by those skilled in the art of the technology to which the present invention pertains. The terms as used herein, unless otherwise explicitly defined, are to be construed as having meanings consistent with those in the specification of the present invention and in related technical fields, and shall not be construed as being idealized or as being interpreted as excessively formal meanings.

### <4. Examples of Experiments>

The present invention is described in further detail with reference to examples of experiments. It is, however, noted that the present invention can be embodied in various modes and shall not be construed as being limited to the examples described below.

Examples 1 to 5 below show cases of allogeneic heterotopic transplantation of a rat liver with the device and method of the present invention. Example 6 below shows a case of allogeneic heterotopic transplantation of a pig liver with the device and method of the present invention. While these examples show cases in which the device and method of the present invention are used to transplant a liver graft into a recipient, it is clear from the disclosure of the specification of the present application that the device of the present invention is also applicable to cases of removing a liver graft from a donor animal.

### <4-1. Example 1: Allogeneic Heterotopic Transplantation of Rat Liver Using Present Invention>

The following experiments aim to transplant a removed rat liver into another individual with the method of the present invention and to assess the hepatic function in vivo. Example 1 describes the detailed procedure of allogeneic heterotopic transplantation of a rat liver using the present invention.

The following animals are used as materials.

Donor Animal: Wister strain; Sex, male
Recipient Animal: Wister strain; Sex, male

Each process including removal of a donor liver, preparation of a liver graft, and transplantation of a liver graft into a recipient is described hereinafter in detail.

### <<A. Removal of Donor Liver>>

A liver graft was removed from a donor animal by the following procedure. (1) Physiological saline was cooled on ice in advance. (2) A donor rat was anesthetized with an anesthesia inhaler (3% isoflurane at 400 ml/min). (3) The abdominal hair of the donor rat was shaved, and laparotomy was performed. (4) The connective membrane was sectioned at midline, and the left phrenic vein was ligated. (5) The infrahepatic inferior vena cava and the right renal vein were isolated. (6) The common hepatic artery was detached to the aorta, and a loop was put around the aorta. (7) After injection of 40 units of heparin from the penile vein, the abdominal aorta was cannulated. (8) The thoracic aorta was blocked with forceps, and the thoracic vena cava was half-cut and flushed with cooled physiological saline. (9) The infrahepatic inferior vena cava was incised below the left renal vein, and the right dorsal connective tissue of the liver was sectioned. (10) The right suprarenal vein was ligated and dissected, and the vena cava was sectioned just above the right renal vein. (11) Two branches except for the proper hepatic artery and one branch of the aorta were ligated, and the loop of the aorta was ligated. (12) A stent was inserted in the bile duct and dissected from the trunk side. (13) The splenic vein and the pyloric vein was ligated and dissected in this order, and the portal vein was sectioned just below the splenic vein. (14) The connective membrane around the stomach and esophagus was sectioned, and after the liver was removed, the vascular plexus between the liver and the esophagus was ligated and dissected. (15) The suprahepatic inferior vena cava at the side of thoracic cavity was isolated with the diaphragm from the adjacent tissue. (16) The aorta was dissected, and the liver was isolated and then preserved in the cooled physiological saline.

### <<B. Preparation of Liver Graft>>

Preparation for transplanting a removed liver graft into a recipient was made by the following procedure. (1) Perfusate (L-15 + RBC: 500 ml) for perfusion of a liver graft was prepared. (2) A transplant-aimed perfusion tube (for portal vein) was connected to the portal vein cannula inserted in the portal vein of the liver graft. (3) The diaphragm was dissected around the suprahepatic inferior vena cava (SHIVC). (4) The infrahepatic inferior vena cava (IHIVC) was dissected near the ligated area, and was ligated and fixed after Out cannulas for perfusate (a cannula for the suprahepatic inferior vena cava and a cannula for infrahepatic inferior vena cava) were inserted therein. (5) A transplant-aimed perfusion tube (for artery) was connected via the cannula for the hepatic artery to the hepatic artery and ligated and fixed. (6) Perfusion was performed by allowing perfusate to enter from the cannula inserted in the portal vein and the cannula inserted in the hepatic artery and allowing perfusate to drain off from the cannula inserted in the suprahepatic inferior vena cava and the cannula inserted in the infrahepatic inferior vena cava. Note that the perfusion may be started at the stage in which at least one or more cannulas were inserted in the liver graft, or may be started after all the cannulas were inserted. The insertion of each cannula and the perfusion may be performed in parallel with the process of removing a liver from a donor.

### <<C. Transplantation of Liver Graft, Perfused by Method of Present Invention, into Recipient>>

A perfused liver graft was transplanted into a recipient by the following procedure. (1) A recipient rat was anesthetized with an anesthesia inhaler (3% isoflurane at 200 ml/min). (2) The concentration was set at 2% isoflurane, and the abdominal hair of the recipient rat was shaved. (3) The skin and peritoneum of the recipient rat was incised to perform laparotomy. (4) The field around the right kidney was exposed to secure the sight.
(5) The infrahepatic inferior vena cava was stripped from the liver to just below the right renal vein. (6) The right renal artery was exposed, and after being stripped from the right renal vein, clipped and sectioned to install a cuff. (7) The infrahepatic inferior vena cava was half-clamped to section the right renal vein and remove the right kidney. (8) A piece of gauze for placing a liver was placed at the right flank. (9) A donor liver was placed so as not to twist the portal vein cannula and the Out cannulas (the cannula for the suprahepatic inferior vena cava and the cannula for the infrahepatic inferior vena cava) (start of dual-flow transplantation). (10) End-to-end anastomosis was performed with the right renal vein of the recipient and the infrahepatic inferior vena cava of the donor. (11) The right renal artery of the recipient was anastomosed to the hepatic artery of the donor by cuffing. (12) The Out cannula (cannula for the suprahepatic inferior vena cava) was pulled out of the donor liver, and the portal vein of the donor liver was clamped simultaneously with the ligation of the suprahepatic inferior vena cava (SHIVC). (13) The clamp was taken off from the hepatic artery, and blood perfusion was performed by the route from the hepatic artery to the infrahepatic inferior vena cava. (14) The transplant-aimed perfusion tube for portal vein was dissected, and the portal vein of the recipient was clamped. (15) End-to-side anastomosis was performed for the portal veins of the recipient and the donor. (16) The clamp was released from the portal vein, and blood reperfusion was performed (completion of dual-flow transplantation). (17) A 2-ml transfusion was injected from the penile vein. (18) A bile duct stent was inserted in the jejunum and sutured. (19) The intestine was put back into the abdominal cavity and washed with warm physiological saline. (20) The peritoneum and the skin were sutured to close the abdomen. The rat was left with heat conditioning until recovery.

### <4-2. Example 2: Effects of Low-Temperature Perfusion and Supplement of Erythrocytes on Liver Graft>

Experiments were conducted to assess a disorder suppressing effect achieved by temperature control and a disorder suppressing effect achieved by supplement of erythrocytes to perfusate when the liver graft was removed and perfused with the device and method of the present invention.

### <<Experimental Method and Results>>

The portal vein and the suprahepatic inferior vena cava were cannulated in Wister rats of 150 to 200 g anesthetized with pentobarbital, and their livers were removed while being perfused by the method of the present invention. Perfusion was continued while the liver was placed in the culture container and floated in culture medium. A sample of the culture medium was taken every four hours, and after the supernatant was collected by centrifugation, it was preserved as an analysis-intended sample in 4°C. After completion of the culture, the analysis-intended sample collected was measured its absorbance at 555 nm with a Wako Transaminase CII-test Wako.

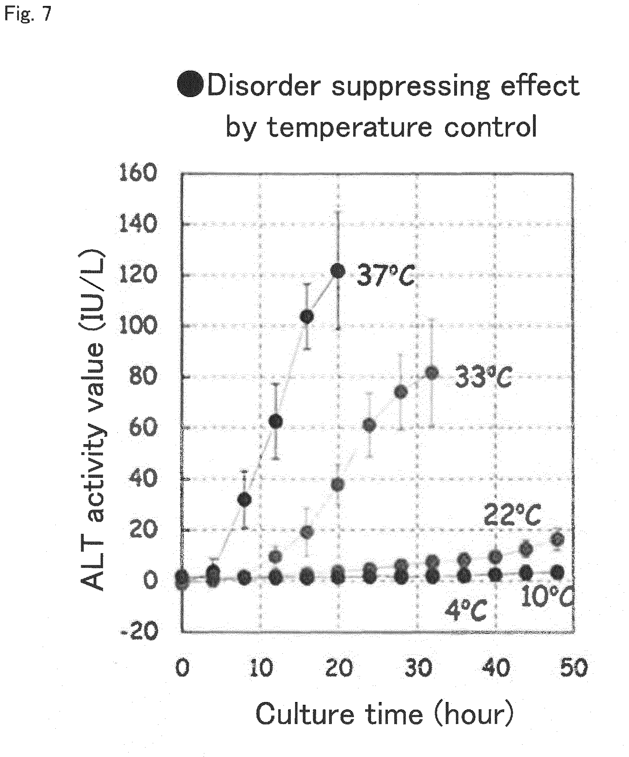

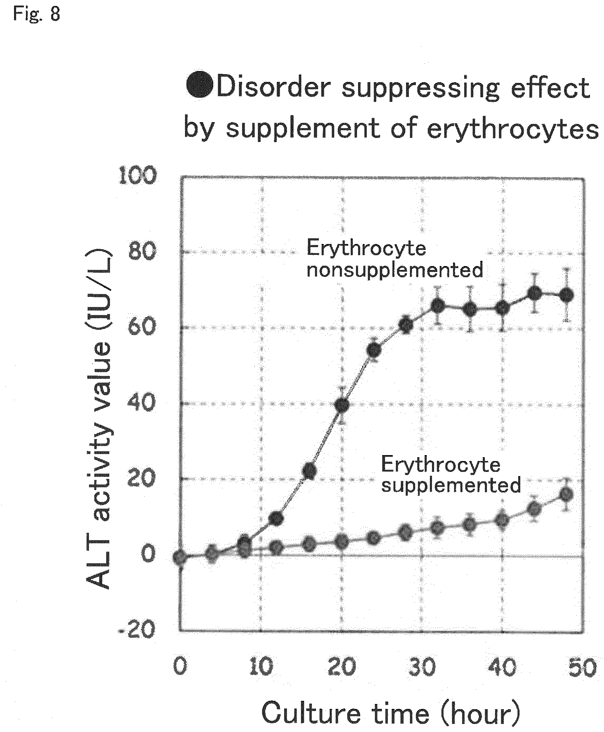

Figs. 7 and 8 show the results of experiments. Fig. 7 illustrates the results of experiments conducted to assess the disorder suppressing effect achieved by temperature control. Specifically, Fig. 7 is a graph showing the relationship between the culture time and a liver disorder marker (ALT activity value) when the temperature during perfusion and culture was set to 37°C, 33°C, 22°C, 10°C, and 4°C. Note that erythrocytes were supplemented to the perfusate at the concentration of 5 × 10¹¹ cells/L. As shown in Fig. 7, for the liver perfused and cultured at temperatures of 37°C and 33°C, elevation of the liver disorder marker was confirmed at a relatively early stage of the culture. On the other hand, for the liver cultured at a temperature zone of 22°C or below, elevation of the liver disorder marker was rarely found.

Fig. 8 illustrates the results of experiments conducted to assess the disorder suppressing effect achieved by the supplement of erythrocytes to the perfusate. Specifically, Fig. 8 is a graph showing the relationship between the culture time and the liver disorder marker (ALT activity value) when erythrocytes were supplemented to the perfusate at a concentration of 5 × 10¹¹ cells/L and when erythrocytes were not supplemented to the perfusate. Note that the temperature during perfusion and culture was set to 22°C. As shown in Fig. 8, elevation of the liver disorder marker was confirmed when erythrocytes were not supplemented, even though perfusion and culture were performed at the same temperature of 22°C. On the other hand, elevation of the liver disorder marker was rarely found when erythrocytes were supplemented. This makes it clear that the elevation of the liver disorder marker can be suppressed by supplementing erythrocytes at a concentration of 5 × 10¹¹ cells/L.

### <4-3. Example 3: Post-Transplant Survival Rate Achieved by Low-Temperature Perfused-Organ Preservation>

### <<Experimental Method and Results>>

To confirm that the liver perfused with the device and method of the present invention can maintain its sufficient function in vivo, a liver graft perfused with perfusate whose erythrocyte concentration was set at 5 × 10¹¹ cells/L under a temperature condition of 22°C was heterotopically transplanted into a living recipient's body. As a control, a liver under 24-hour simple cooling preservation with UW solution and a liver perfused with erythrocyte-nonsupplemented L-15 medium under a temperature condition of 22°C were transplanted into recipients.

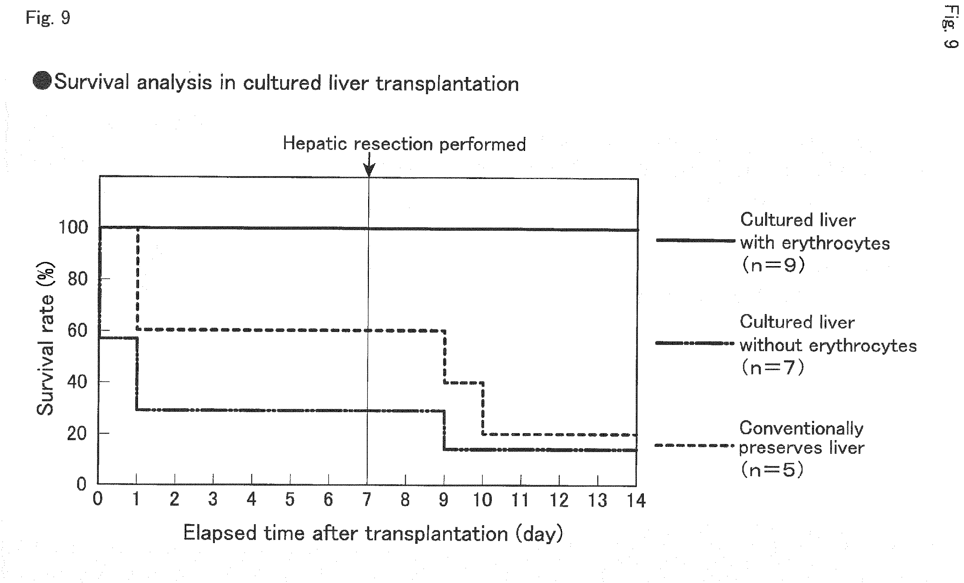

Fig. 9 shows the experimental results. Note that the "cultured liver" in Fig. 9 refers to a liver perfused by the method of the present invention. Also, the "conventionally preserved liver" refers to a liver that has undergone 24-hour simple cooling preservation with UW solution. Specifically, a group of recipients with livers transplanted after 24-hour simple cooling preservation with UW solution (conventionally preserved liver) is indicated by the dashed line in Fig. 9. A group of recipients with livers transplanted after perfusion with erythrocyte-nonsupplemented L-15 medium under a temperature condition of 22°C (cultured liver without erythrocytes) is indicated by the double-dot-dash line. Moreover, a group of recipients with livers transplanted after perfusion with erythrocyte-supplemented L-15 medium under a temperature condition of 22°C (cultured liver with erythrocytes) is indicated by the solid line.

For these three groups, follow-up observation was first performed for one week after transplantation (elapsed time of seven days from transplantation). As shown in Fig. 9, the group of recipients with livers transplanted after 24-hour simple cooling preservation with UW solution and the group of recipients with livers transplanted after perfusion with erythrocyte-nonsupplemented L-15 medium under a temperature condition of 22°C had relatively low survival rates. Specifically speaking, the group of recipients with livers transplanted after 24-hour simple cooling preservation with UW solution had a survival rate of 100% on the day of transplantation. However, the survival rate dropped to 60% one day after transplantation. The group of recipients with livers transplanted after perfusion with erythrocyte-nonsupplemented L-15 medium under a temperature condition of 22°C had a survival rate of approximately 57% on the day of transplantation. This survival rate dropped to approximately 29% one day after transplantation. On the other hand, the group of recipients with livers transplanted into a living body after 24-hour culture with erythrocyte-supplemented L-15 medium had a survival rate of 100%.

After one-week post-transplant follow-up observation (elapsed time of seven days after transplantation), for individuals for whom organ engraftment was observed, partial hepatectomy was performed on the recipient's original liver and the portal blood was switched on the day that was seven days after transplantation. Then, follow-up observation was performed for another week. The survival rate of the group of recipients with livers transplanted after 24-hour simple cooling preservation with UW solution dropped from 60% to 40% nine days after transplantation and further dropped to 20% ten days after transplantation. Also, the survival rate of the group of recipients with livers transplanted after perfusion with erythrocyte-nonsupplemented L-15 medium under a temperature condition of 22°C dropped from approximately 29% to approximately 14% nine days after transplantation. On the other hand, the survival rate of the group of recipients with livers transplanted after 24-hour culture with erythrocyte-supplemented L-15 medium remained 100% until 14 days after transplantation.

Furthermore, in the group of recipients with livers transplanted after 24-hour culture with erythrocyte-supplemented L-15 medium, the transplanted cultured liver regenerated to reach the weight of the original liver of the individual, and the liver preserved by perfusion and culture retained its essential function for individual survival even after transplantation into a living body. In other words, the liver graft perfused by the method of the present invention was found to have a liver regenerative ability capable of covering decreased hepatic function of the recipient as an individual.

### <4-4. Example 4: Resuscitation of Liver Donated from Cardiac Arrest Donor with Low-Temperature Perfusion>

A liver injured by severe warm ischemia was cultured ex vivo with the device and method of the present invention in order to show that the device and method of the present invention were also available for a liver donated from a cardiac arrest donor (donation after cardiac death (DCD) donor).

### <<Experimental Method and Results>>

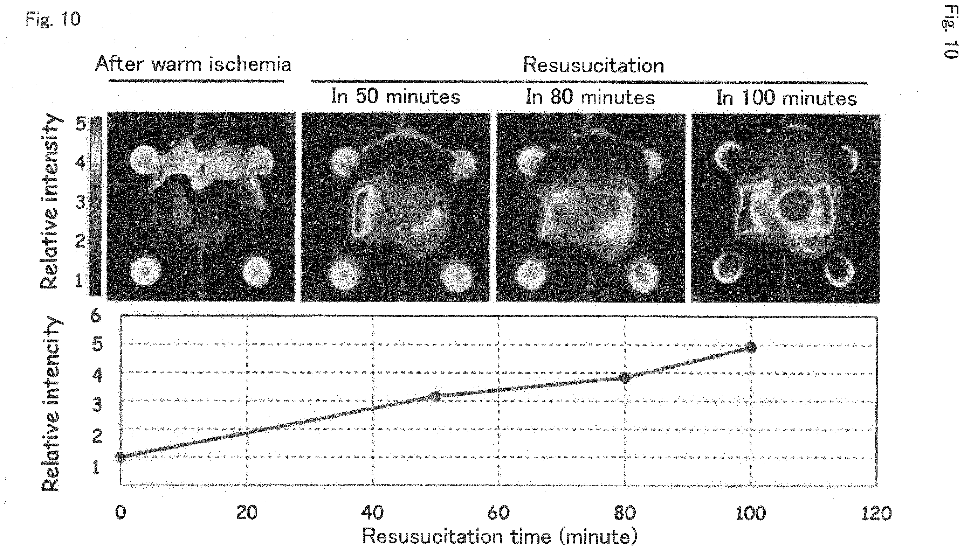

A liver was removed from a luciferase-expressing rat under cardiac arrest, and after 90-minute warm ischemia from cardiac arrest, 100-minute ex vivo perfusion (resuscitation) was performed with the device and method of the present invention. Note that the 100-minute ex vivo perfusion was performed with perfusate (L-15 medium) where the erythrocyte concentration was set at 5 × 10¹¹ cells/L under a temperature condition of 22°C. Fig. 10 shows the relative intensity of ATP of the removed liver. The upper section in Fig. 10 gives diagrams showing that the ATP concentration of the liver was converted into the relative intensity indicating a numerical range from zero to five. The lower section in Fig. 10 shows the average of the relative intensity of each designated part of the liver.

As shown in Fig. 10, the relative intensity of ATP was approximately 1.0 for the liver after warm ischemia. With the ex vivo perfusion (resuscitation) using the perfusion device of the present invention, the relative intensity of ATP reached approximately 3.1 after 50 minutes, reached approximately 3.9 after 80 minutes, and reached approximately 4.9 after 100 minutes. In other words, the recovery of ATP consumed during ischemic treatment was confirmed. This indicates that the device and method of the present invention are capable of recovering the energy metabolic state of the liver while suppressing the disorder of the ischemic liver.

### <4-5. Example 5: Post-Transplant Survival Rate of Resuscitated Organ>

An ischemic liver perfused ex vivo (resuscitation) by the method described in Example 4 was transplanted into a recipient, and follow-up observation was performed after the transplantation.

### <<Experimental Method and Results>>

With the method described in Example 4, a liver that had been in a warm ischemia for 90 minutes after cardiac arrest and then cultured ex vivo for 100 minutes with perfusate (L-15 medium) whose erythrocyte concentration was set at 5 × 10¹¹ cells/L under a temperature condition of 22°C was transplanted into a living recipient's body. As a control, a liver that has undergone simple cooling preservation with UW solution and a liver resuscitated with erythrocyte-nonsupplemented perfusate (L-15 medium) were transplanted into recipients. Similarly to Example 3, for individuals for whom organ engraftment was observed one week after transplantation, partial hepatectomy was performed on the recipient original liver 9, and the portal blood was switched.

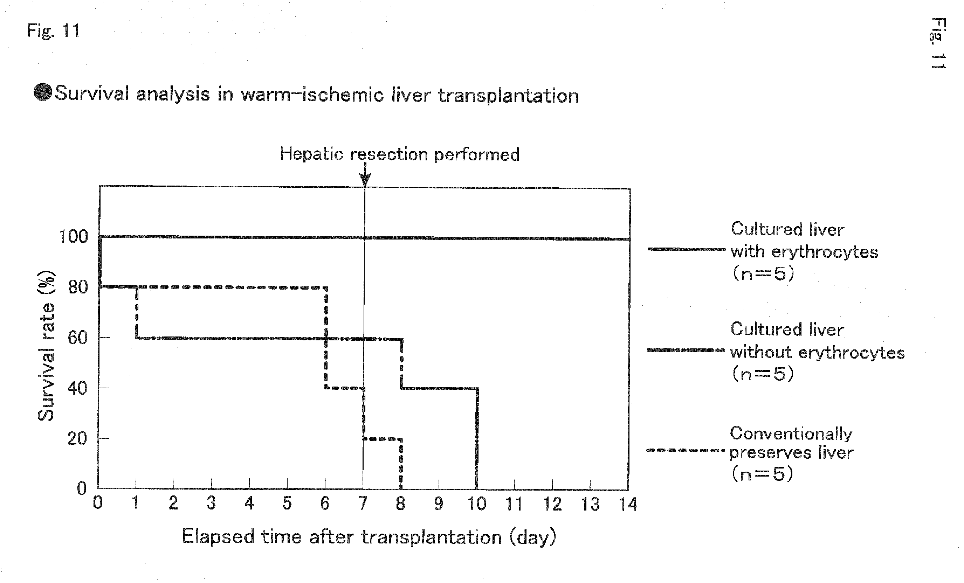

Fig. 11 illustrates experimental results. Note that the "resuscitated liver" in Fig. 11 refers to an ischemic liver resuscitated by the method of the present invention. Also, the "conventionally preserved liver" refers to an ischemic liver preserved at low temperature for 100 minutes with UW solution. Specifically, a group of recipients with livers transplanted after 100-minute simple cooling preservation with UW solution (conventionally preserved liver) is indicated by the dashed line in Fig. 11. A group of recipients with livers transplanted after resuscitation with erythrocyte-nonsupplemented perfusate (L-15 medium) (resuscitated liver without erythrocytes) is indicated by the double-dot-dash line. Also, a group of recipients with livers transplanted after resuscitation with erythrocyte-supplemented perfusate (L-15 medium) (resuscitated liver with erythrocytes) is indicated by the solid line.

For these three groups, follow-up observation was performed. As shown in Fig. 11, the group of recipients with livers transplanted after resuscitation with UW solution had a survival rate of 80% on the day of transplantation, and the survival rate dropped to 40% six days after transplantation. The group of recipients with livers transplanted after resuscitation with the erythrocyte-nonsupplemented perfusate (L-15 medium) had a survival rate of 80% on the day of transplantation, and the survival rate dropped to 60% one day after transplantation. On the other hand, the survival rate of the group with livers transplanted after resuscitation with erythrocyte-supplemented perfusate (L-15 medium) remained 100% until seven days after transplantation. As shown here, during one week after transplantation (elapsed time of seven days after transplantation), the recipients with ischemic livers transplanted after resuscitation using the device and method of the present invention exhibited a relatively high survival rate.

However, the survival rates of the group of recipients with livers transplanted after resuscitation with UW solution and the group of recipients with livers transplanted after resuscitation with erythrocyte-nonsupplemented perfusate (L-15 medium) dropped after the partial hepatectomy of the recipients' livers. Specifically, the survival rate of the group of recipients with livers transplanted after resuscitation with UW solution dropped to 20% on the day of partial hepatectomy that was seven days after transplantation, and the survival rate reached 0% eight days after transplantation. Moreover, the survival rate of the group with livers transplanted after resuscitation with erythrocyte-nonsupplemented perfusate (L-15 medium) dropped to 40% eight days after transplantation and reached 0% 10 days after transplantation. On the other hand, the survival rate the group of recipients with ischemic livers transplanted after ex vivo culture with erythrocyte-supplemented perfusate (L-15 medium) remained 100% until 14 days after transplantation.

This indicates that ischemic livers that had been considered incompatible for transplantation become transplantable by using the device and method of the present invention.

### <4-6. Example 6: Allogeneic Heterotopic Transplantation of Pig Liver Using Present Invention>

Experiments were conducted to assess whether a pig liver can be engrafted when the liver graft is removed and perfused with the device and method of the present invention.

### <<Experimental Method and Results>>

A specific experimental procedure is almost the same as the procedure of experiments conducted on rats described in Example 1. The differences in procedure between the experiments on pigs in Example 6 and the experiments on rats in Example 1 are as follows. First, in Example 1, Out cannulas for perfusate were connected to both of the infrahepatic inferior vena cava and the suprahepatic inferior vena cava from after the removal of the donor liver until the transplantation into the recipient. However, in Example 6, no cannula was connected to the infrahepatic inferior vena cava and an Out cannula was connected to only the suprahepatic inferior vena cava. In Example 1, a piece of gauze was placed where the donor liver was to be put, but in Example 6, no gauzes were placed and the donor liver was placed directly inside the abdominal cavity of the recipient. Moreover, in Example 1, the right kidney was removed, but in Example 6, no kidney was removed. Therefore, in Example 1, end-to-end anastomosis was performed with the infrahepatic inferior vena cava of the donor and the right renal vein of the recipient, but in Example 6, end-to-side anastomosis was performed with the infrahepatic inferior vena cava of the donor and the right renal vein of the recipient. The other procedure was the same.

In this experiment, after the removal of the donor liver, mechanical perfusion preservation was performed for 90 minutes with the device and method of the present invention. As perfusate, FBS, heparin, and erythrocyte-supplemented L-15 medium were used. The flow rate of the perfusate was set at 0.5 ml/min/g. Then, the donor liver was placed in the recipient's body (start of transplantation). Rinsing and anastomosis of the blood vessels were performed over a period of approximately 60 minutes. As a rinse solution, erythrocyte-supplemented L-15 medium was used. The flow rate of the rinse solution was set at 0.5 ml/min/g. After the anastomosis of the blood vessels, the blood was reperfused (completion of transplantation).

One experiment conducted showed that the consciousness of the recipient recovered, and engraftment succeeded. This confirmed that dual-flow transplantation was effective not only for rats but also for pigs.

As described above, in Example 6, two-in-one-out perfusion was performed in which the donor liver was perfused with two perfusate inflow cannulas and one perfusate outflow cannula (Out cannula) from after the removal of the donor liver until the transplantation into the recipient.

Here, the hepatic artery and the portal vein used for the inflow of perfusate supply perfusate to the liver from separate parts of the liver. Thus, the supply of perfusate from both of the hepatic artery and the portal vein allows the perfusate to be supplied more thoroughly in the liver than the supply of perfusate from only one of the hepatic artery and the portal vein. This suppresses the onset of disorder in the liver during the perfusion process, leading to a higher possibility of recovery in liver function.

Whereas, when the perfusate drains off from both of the infrahepatic inferior vena cava and the suprahepatic inferior vena cava, the efficiency of perfusate drainage improves slightly, as compared with when the perfusate drains off from only one of the infrahepatic inferior vena cava and the suprahepatic inferior vena cava. However, the infrahepatic inferior vena cava and the suprahepatic inferior vena cava are both part of a single blood vessel passing through the liver. Thus, the rate of improvement in drainage efficiency is small even if the perfusate drains off from only one of the infrahepatic inferior vena cava and the suprahepatic inferior vena cava, as compared with when the perfusate drains off from both of the infrahepatic inferior vena cava and the suprahepatic inferior vena cava.

For these reasons, connecting the two perfusate inflow cannulas to the liver contributes greatly to suppressing disorder in the liver and improving the possibility of functional recovery in dual-flow perfusion. Therefore, in the perfusion treatment of the liver, the onset of liver disorder during perfusion and preservation can be suppressed effectively even in the two-in-one-out perfusion as in the dual-flow perfusion using two perfusate inflow cannulas and two perfusate outflow cannulas, as compared with the single flow perfusion using one perfusate inflow cannula and one perfusate outflow cannula. Furthermore, the function of the liver can recover during perfusion and preservation.

Here, the procedure for removing a donor liver and the procedure for transplanting a removed liver into a recipient, both procedures using two-in-one-out perfusion, are described hereinafter with reference to Figs. 14 and 15. To perform two-in-one-out perfusion, the above-described perfusion device 1 for dual-flow perfusion may be employed, or a perfusion device that replaces the two perfusate outflow pathways 40 of the perfusion device 1 with one outflow pathway may be employed.

Fig. 14 is a flowchart showing the procedure of the process of removing a liver graft from a donor and performing two-in-one-out perfusion with the perfusion device 1. In step (1B), one of the hepatic artery and the portal vein of the donor is incised or sectioned (step (11B)). Then, a first perfusate inflow cannula 32 is connected to the incised or sectioned area (step (12B)). Next, the suprahepatic inferior vena cava of the donor is incised or sectioned (step (13B)). Then, a perfusate outflow cannula 42 is connected to the incised or sectioned area (step (14B)).

Next, in step (2B), perfusate is allowed to enter from the first perfusate inflow cannula 32, and perfusate is allowed to drain off from the first perfusate outflow cannula 42. This starts perfusion of the liver graft 9 with perfusate. Accordingly, single flow perfusion is started with one route of perfusate inflow pathway 30 and one route of perfusate outflow pathway 40.

Subsequently, in step (3B), the other of the hepatic artery and the portal vein that has not been incised or sectioned in advance in step (1B) is incised or sectioned (step (31B)). Then, a second perfusate inflow cannula 32 is connected to the incised or sectioned area (step (32B)). Subsequently, the other of the suprahepatic inferior vena cava and the infrahepatic inferior vena cava that has not been incised or sectioned in step (1B) is clipped and sectioned (step (33B)). Here, a cannula is not inserted in this blood vessel.

Thereafter, in step (4B), perfusate is allowed to enter from the first and second perfusate inflow cannulas 32, and perfusate is allowed to drain off from the perfusate outflow cannula 42. In other words, two-in-one-out perfusion starts (step (41B)). Then, the liver 9 is removed from the donor while the perfusion of the liver graft 9 with perfusate is maintained (step (42B)).

In step (1B) described above, the combination of blood vessels that are incised or sectioned (i.e., the combination of blood vessels that are incised or sectioned in advance) is not limited and can be appropriately selected by those skilled in the art in consideration of conditions such as the volume of the bloodstream of each blood vessel and organ status. Any combinations are allowed, for example, a combination of the hepatic artery and the suprahepatic inferior vena cava, a combination of the hepatic artery and the infrahepatic inferior vena cava, a combination of the portal vein and the suprahepatic inferior vena cava, and a combination of the portal vein and the infrahepatic inferior vena cava.

For example, when orthotopic transplantation is performed, the perfusate outflow cannula 42 is connected to the infrahepatic inferior vena cava in step (1B), and the suprahepatic inferior vena cava that is not connected to the perfusate outflow cannula 42 is anastomosed to a recipient's blood vessel in step (6B) described below. Whereas, when heterotopic transplantation is performed, the perfusate outflow cannula 42 is connected to the suprahepatic inferior vena cava in step (1B), and the infrahepatic inferior vena cava that is not connected to the perfusate outflow cannula 42 is anastomosed to a recipient's blood vessel in step (6B) described below. In this way, the blood vessel that is selected and connected to the perfusate outflow cannula 42 at the time of removal of the donor liver is not the one that is anastomosed in advance to the recipient's blood vessel at the time of transplantation.

For each combination, the order of blood vessels to be incised or sectioned is not limited and can be appropriately selected by those skilled in the art in consideration of conditions such as the volume of the bloodstream of each blood vessel and organ status. In step (1B), step (12B) needs to be performed at least after step (11B), and step (14B) needs to be performed at least after step (13B). In step (3B), step (32B) needs to be performed at least after step (31B). Also, step (41B) may be performed after step (32B) and after step (33B).

In steps (1B) to (4B) described above, treatments normally performed in the procedure for removing the liver graft 9 from a donor (for example, excision of connective tissues, peeling of blood vessels, temporary ligation or clamping of blood vessels for section of blood vessel, blockage and dissection of the bile duct, application of coagulation agents to the liver graft 9, hemostasis for operated sites) can be conducted as needed by those skilled in the art.

In step (1B) described above, one of the hepatic artery and the portal vein of the donor and one of the suprahepatic inferior vena cava and the infrahepatic inferior vena cava are connected to the cannulas while the blood flow is maintained in the other of the hepatic artery and the portal vein of the donor and the other of the suprahepatic inferior vena cava and the infrahepatic inferior vena cava. In this way, the liver 9 to be removed can be protected from being in an ischemic state during the process of connecting one of the hepatic artery and the portal vein of the donor and one of the suprahepatic inferior vena cava and the infrahepatic inferior vena cava to the cannulas. This suppresses the onset of disorder in the liver 9 to be removed.

In step (3B) described above, single flow perfusion is performed with one of the hepatic artery and the portal vein of the donor and one of the suprahepatic inferior vena cava and the infrahepatic inferior vena cava. Therefore, in step (3B), the liver 9 to be removed can be protected from being in an ischemic state during the process of connecting the other of the hepatic artery and the portal vein of the donor and the other of the suprahepatic inferior vena cava and the infrahepatic inferior vena cava to the cannulas. This further suppresses the onset of disorder in the liver 9 to be removed.

Fig. 15 is a flowchart showing the procedure of the process of transplanting the liver graft 9 into a recipient with the perfusion device 1. First, in step (5B), the liver graft 9 is prepared, with the perfusate inflow cannulas 32 connected to either the portal vein and the hepatic artery and the perfusate outflow cannula 42 connected to either the suprahepatic inferior vena cava or the infrahepatic inferior vena cava, and with perfusate allowed to enter from the two perfusate inflow cannulas 32 and perfusate allowed to drain off from the perfusate outflow cannula 42. In other words, the liver 9 under two-in-one-out perfusion is prepared.

Next, in step (6B), the perfusate inflow cannulas 32 is extracted from one of the portal vein and the hepatic artery of the liver graft 9 (step (61B)). Then, the blood vessel from which the perfusate inflow cannula 32 has been extracted is anastomosed to a corresponding blood vessel of the recipient while the perfusion of the liver graft 9 with perfusate is maintained (step (62B)). Also, one of the suprahepatic inferior vena cava and the infrahepatic inferior vena cava, the blood vessel in which the perfusate outflow cannula 42 is not inserted is anastomosed to a corresponding blood vessel of the recipient (step (63B)). In this way, single flow perfusion is performed with the unextracted perfusate inflow cannula 32 and the unextracted perfusate outflow cannula 42 during the process of step (6B). This prevents the liver graft 9 from being in an ischemic state in step (6B).

Next, in step (7B), the unextracted perfusate inflow cannula 32 is extracted from the other of the portal vein and the hepatic artery of the liver graft 9 (step (71B)). Also, the unextracted perfusate outflow cannulas 42 is extracted from the other of the suprahepatic inferior vena cava and the infrahepatic inferior vena cava of the liver graft 9 (step (72B)). Then, the blood vessel from which the perfusate inflow cannula 32 has been extracted is anastomosed to a corresponding blood vessel of the recipient (step (73B)). Also, the blood vessel from which the perfusate outflow cannula 42 has been extracted is anastomosed to a corresponding blood vessel of the recipient (step (74B)). Note that the blood vessel from which the perfusate outflow cannula 42 has been extracted in step (73B) may be ligated in step (74B).

In step (6B) described above, the combination of blood vessels of the donor liver 9 for use in anastomosis (i.e., the combination of blood vessels that are anastomosed in advance) is not limited and can be appropriately selected by those skilled in the art in consideration of conditions such as the volume of the bloodstream of each blood vessel and organ status. Any combinations are allowed, for example, a combination of the hepatic artery and the suprahepatic inferior vena cava, a combination of the hepatic artery and the infrahepatic inferior vena cava, a combination of the portal vein and the suprahepatic inferior vena cava, and a combination of the portal vein and the infrahepatic inferior vena cava. For each combination, the order of blood vessels to be anastomosed is not limited and can be appropriately selected by those skilled in the art in consideration of conditions such as the volume of the bloodstream of each blood vessel and organ status. For this reason, the definition of "one" and "the other" in steps (1B) to (4B) may be the same as or different from the definition of "one" and "the other" in steps (5B) to (7B).

The recipient's blood vessels that are anastomosed to the blood vessels of the donor liver 9 may be the same type of blood vessels as the donor's blood vessels used for anastomosis, or may be a different type of blood vessels. For example, in the orthotopic transplantation of the liver 9, the recipient's blood vessels that are anastomosed to the blood vessels of the donor liver 9 may be the same type of blood vessels as the blood vessels of the donor liver 9 used for anastomosis. Furthermore, in the heterotopic transplantation of the liver 9, the recipient's blood vessels that are anastomosed to the blood vessels of the donor liver 9 may be a different type of blood vessels from the blood vessels of the donor liver 9 used for anastomosis. The blood vessel type of the recipient used in the heterotopic transplantation of the liver 9 can be appropriately selected based on common general technical knowledge by those skilled in the art.

In steps (5B) to (7B) described above, treatments normally performed in the procedure for transplanting the liver graft 9 into a recipient (for example, excision of connective tissues, peeling of blood vessels, temporary ligation or clamping of blood vessels for section and anastomose of the vessels, anastomosis of blood vessels, anastomosis of the bile duct, hemostasis for operated sites) can be conducted as needed by those skilled in the art.

In step (6B) described above, one of the hepatic artery and the portal vein of the donor and one of the suprahepatic inferior vena cava and the infrahepatic inferior vena cava of the donor are anastomosed in advance to the recipient's blood vessels. Even during the process of anastomosing these two blood vessels, single flow perfusion is maintained in the other of the hepatic artery and the portal vein and the other of the suprahepatic inferior vena cava and the infrahepatic inferior vena cava. In this way, the liver graft 9 can be protected from being in an ischemic state during transplantation surgery. Thus, it is possible to provide ample time to anastomose two blood vessels that are sutured in advance with the recipient's blood vessels.

### Reference Signs List

- 1: Perfusion device
- 9: Liver
- 10: Bioreactor area
- 20: Perfusate reservoir
- 21: Temperature control system
- 22: Gas exchange system
- 30: Perfusate inflow pathway
- 31: Pipe
- 32: Perfusate inflow cannula
- 33: Pump
- 34: Temperature control unit
- 35: Degassing unit
- 36: Manometer
- 37: Flowmeter
- 40: Perfusate outflow pathway
- 41: Pipe
- 42: Perfusate outflow cannula
- 43: Pump
- 44: Manometer
- 45: Flowmeter

## Claims

1. A perfusion device for a liver graft and for use in removing a liver graft from a donor or for use in transplanting a liver graft into a recipient,
said device comprising the following (A) to (D):
(A) cannulas (i) to (iv) including:
(i) a perfusate inflow cannula connected to a portal vein of said liver graft;
(ii) a perfusate inflow cannula connected to a hepatic artery of said liver graft;
(iii) a perfusate outflow cannula connected to a suprahepatic inferior vena cava of said liver graft; and
(iv) a perfusate outflow cannula connected to an infrahepatic inferior vena cava of said liver graft;
(B) one or a plurality of perfusate containers containing perfusate;
(C) one or a plurality of pumps; and
(D) a pipe
wherein said (A) to (D) are configured such that, when said liver graft is perfused, at least one of said one or a plurality of pumps allows the perfusate contained in at least one of said one or a plurality of perfusate containers to enter said liver graft from said perfusate inflow cannula (i) and/or said perfusate inflow cannula (ii) through said pipe, and allows the perfusate in said liver graft to drain off from said perfusate outflow cannula (iii) and/or said perfusate outflow cannula (iv).

2. The perfusion device according to claim 1, wherein
when said liver graft is perfused, said perfusate inflow cannula (i) and/or said perfusate inflow cannula (ii), said liver graft, said perfusate outflow cannula (iii) and/or said perfusate outflow cannula (iv), and said perfusate containers form a perfusion circuit through said pipe.

3. The perfusion device according to claim 1 or 2, wherein
when said device is used to remove a liver graft from a donor, said device is used in a method comprising the following steps (1) to (4) of:
(1) incising or sectioning one of the hepatic artery and the portal vein of the donor to connect first one of said perfusate inflow cannulas to an incised or sectioned area, and incising or sectioning one of the suprahepatic inferior vena cava and the infrahepatic inferior vena cava of the donor to connect first one of said perfusate outflow cannulas to an incised area or an area;
(2) allowing perfusate to enter from said first one of said perfusate inflow cannulas and allowing perfusate to drain off from said first one of said perfusate outflow cannulas to start perfusion of said liver graft with perfusate;
(3) incising or sectioning the other of the hepatic artery and the portal vein, the other having not been incised or sectioned in step (1), to connect second one of said perfusate inflow cannulas to an incised or sectioned area, and incising or sectioning the other of the suprahepatic inferior vena cava and the infrahepatic inferior vena cava, the other having not been incised or sectioned in step (1), to connect second one of said perfusate outflow cannulas to an incised or sectioned area; and
(4) allowing perfusate to enter from said first and second ones of said perfusate inflow cannulas and allowing perfusate to drain off from said first and second ones of said perfusate outflow cannulas to remove a liver from the donor while maintaining the perfusion of said liver graft with perfusate.

4. The perfusion device according to claim 1 or 2, wherein
when said device is used to transplant a liver graft into a recipient, said device is used in a method comprising the following steps (5) to (7) of:
(5) preparing a liver graft, with said perfusate inflow cannula (i) connected to the portal vein, said perfusate inflow cannula (ii) connected to the hepatic artery, said perfusate outflow cannula (iii) connected to the suprahepatic inferior vena cava, and said perfusate outflow cannula (iv) connected to the infrahepatic inferior vena cava, and with perfusate allowed to enter from said perfusate inflow cannulas (i) and (ii) and perfusate allowed to drain off from said perfusate outflow cannulas (iii) and (iv);
(6) extracting said perfusate inflow cannula from one of the portal vein and the hepatic artery of said liver graft and extracting said perfusate outflow cannula from one of the suprahepatic inferior vena cava and the infrahepatic inferior vena cava of said liver graft, and while maintaining the perfusion of said liver graft with perfusate, anastomosing said blood vessel from which said perfusate inflow cannula has been extracted to a corresponding blood vessel of the recipient and anastomosing said blood vessel from which said perfusate outflow cannula has been extracted to a corresponding blood vessel of the recipient; and
(7) extracting unextracted one of said perfusate inflow cannulas from the other of the portal vein and the hepatic artery of said liver graft, extracting unextracted one of said perfusate outflow cannulas from the other of the suprahepatic inferior vena cava and the infrahepatic inferior vena cava of said liver graft, anastomosing said blood vessel from which said perfusate inflow cannula has been extracted to a corresponding blood vessel of the recipient, and anastomosing said blood vessel from which said perfusate outflow cannula has been extracted to a corresponding blood vessel of the recipient.

5. The perfusion device according to any one of claims 1 to 4, further comprising:
a container capable of immersing at least part of said liver graft in liquid.

6. The perfusion device according to any one of claims 1 to 5, wherein
at least part of said pipe is made of an elastic material or has an elastic structure.

7. The perfusion device according to claim 6, wherein
said elastic structure is a bellows structure.

8. The perfusion device according to any one of claims 1 to 7, wherein
said pipe is provided with a flowmeter that measures a flow rate of perfusate and a manometer that measures a flow pressure of perfusate,
said pump has an operation control system, and
when said liver graft is perfused, said operation control system of said pump operates in accordance with a measured value of said flowmeter and/or a measured value of said manometer to maintain the flow rate and/or flow pressure of perfusate during perfusion within a predetermined range.

9. The perfusion device according to any one of claims 1 to 8, wherein
a degassing unit that eliminates bubbles in perfusate is provided in said pipe that connects said perfusate containers and said perfusate inflow cannula (i) or (ii).

10. The perfusion device according to any one of claims 1 to 9, wherein
said perfusate containers include a temperature control system that regulates a temperature of perfusate and/or a gas exchange system that regulates a volume of dissolved oxygen, dissolved carbon dioxide and/or dissolved nitrogen in perfusate.

11. The perfusion device according to any one of claims 1 to 10, wherein
a temperature control unit that regulates a temperature of perfusate is provided in said pipe that connects said perfusate containers and said perfusate inflow cannula (i) or (ii).

12. The perfusion device according to claim 10 or 11, wherein
when said liver graft is perfused, the temperature of the perfusate that enters said liver graft is controlled in a range of 20 to 25°C by said temperature control system of said perfusate containers and/or said temperature control unit provided in said pipe.

13. The perfusion device according to any one of claims 1 to 12, wherein
when said liver graft is perfused, said perfusate contains an oxygen carrier.

14. The perfusion device according to claim 13, wherein
said oxygen carrier is an erythrocyte.

15. A method of removing a liver graft from a donor, comprising the steps of:
(1) incising or sectioning one of a hepatic artery and a portal vein of a donor to connect a first perfusate inflow cannula to an incised or sectioned area, and incising or sectioning one of a suprahepatic inferior vena cava and an infrahepatic inferior vena cava of the donor to connect a first perfusate outflow cannula to an incised or sectioned area;
(2) allowing perfusate to enter from said first perfusate inflow cannula and allowing perfusate to drain off from said first perfusate outflow cannula to start perfusion of said liver graft with perfusate;
(3) incising or sectioning the other of the hepatic artery and the portal vein, the other having not been incised or sectioned in step (1), to connect a second perfusate inflow cannula to an incised or sectioned area, and incising or sectioning the other of the suprahepatic inferior vena cava and the infrahepatic inferior vena cava, the other having not been incised or sectioned in step (1), to connect a second perfusate outflow cannula to an incised or sectioned area; and
(4) allowing perfusate to enter from said first and second perfusate inflow cannulas and allowing perfusate to drain off from said first and second perfusate outflow cannulas to remove a liver from the donor while maintaining the perfusion of said liver graft with perfusate.

16. The method according to claim 15, wherein
said steps (1) to (4) are performed while the liver of the donor is placed in a range of 20 to 25°C.

17. The method according to claim 15 or 16, wherein
said perfusate used in said steps (1) to (4) contains an oxygen carrier.

18. The method according to claim 17, wherein
said oxygen carrier is an erythrocyte.

19. A method of transplanting a liver graft into a recipient, comprising the steps of:
(5) preparing a liver graft, with first one of perfusate inflow cannulas connected to a portal vein, second one of perfusate inflow cannulas connected to a hepatic artery, first one of perfusate outflow cannulas connected to a suprahepatic inferior vena cava, and second one of perfusate outflow cannulas connected to an infrahepatic inferior vena cava, and with perfusate allowed to enter said first and second ones of perfusate inflow cannulas and perfusate allowed to drain off from said first and second ones of perfusate outflow cannulas; and
(6) extracting said perfusate inflow cannula from one of the portal vein and the hepatic artery of said liver graft and extracting said a perfusate outflow cannula from one of the suprahepatic inferior vena cava and the infrahepatic inferior vena cava of said liver graft, and while maintaining the perfusion of said liver graft with perfusate, anastomosing said blood vessel from which said perfusate inflow cannula has been extracted to a corresponding blood vessel of the recipient and anastomosing said blood vessel from which said perfusate outflow cannula has been extracted to a corresponding blood vessel of the recipient.

20. The method according to claim 19, further comprising the step of:
(7) extracting unextracted one of said perfusate inflow cannulas from the other of the portal vein and the hepatic artery of said liver graft, extracting unextracted one of said perfusate outflow cannulas from the other of the suprahepatic inferior vena cava and the infrahepatic inferior vena cava of said liver graft, anastomosing said blood vessel from which the perfusate inflow cannula has been extracted to a corresponding blood vessel of the recipient, and anastomosing said blood vessel from which the perfusate outflow cannula has been extracted to a corresponding blood vessel of the recipient.

21. The method according to claim 19, further comprising the step of:
(8) extracting unextracted one of said perfusate inflow cannulas from the other of the portal vein and the hepatic artery of said liver graft, extracting unextracted one of said perfusate outflow cannulas from the other of the suprahepatic inferior vena cava and the infrahepatic inferior vena cava of said liver graft, and ligating said blood vessel from which said perfusate inflow cannula has been extracted or anastomosing said blood vessel to a corresponding blood vessel of the recipient, and ligating said blood vessel from which said perfusate outflow cannula has been extracted or anastomosing said blood vessel to a corresponding blood vessel of the recipient.

22. The method according to any one of claims 19 to 21, wherein
each step is performed while said liver graft is placed in a range of 20 to 25°C.

23. The method according to any one of claims 19 to 22, wherein
said perfusate used in each step contains an oxygen carrier.

24. The method according to claim 23, wherein
said oxygen carrier is an erythrocyte.
